# EUROPEAN PATENT APPLICATION

(11) **EP 4 407 029 A1**
(43) Date of publication of application: **31.07.2024**
(21) Application number: 22872206.2
(22) Date of filing: 26.09.2022
(51) Int. Cl.: C12N 15/10, C12P 19/34, C12Q 1/68, C40B 50/06

(54) **METHOD AND KIT FOR NUCLEIC ACID LIBRARY CONSTRUCTION AND SEQUENCING**

(30) Priority: 26.09.2021 CN 202111126861; 26.09.2021 CN 202111127225; 26.09.2021 CN 202111127222; 15.11.2021 CN 202111348934
(71) Applicant: Hangzhou New Horizon Health Technology Co., Ltd., Hangzhou, Zhejiang 310052 (CN)
(72) Inventor: MA, Shiqing, Hangzhou, Zhejiang 310052 (CN); LIU, Jun, Hangzhou, Zhejiang 310052 (CN); CHEN, Yiyou, Hangzhou, Zhejiang 310052 (CN); DING, Kefeng, Hangzhou, Zhejiang 310052 (CN)
(74) Representative: Cooley (UK) LLP
(86) International application number: PCT/CN2022/121311
(87) International publication number: WO 2023/046163

(57) **Abstract**

The present invention relates to a method and kit for removing linker self-linking products during sequencing library construction and for constructing a sequencing library. In particular, provided is a method and kit for the selective cleavage of DNA double strands and DNA single strands, a method and kit for selective cleavage by using DNA-RNA-DNA:cDNA heterozygous chains and DNA:cDNA double strands, and a method and kit for DNA and RNA library co-construction.

## Description

### Technical Field

The present invention relates to the field of biotechnology, and in particular, to the field of gene sequencing. Specifically, the present invention relates to a method and a related kit for removing linker self-linking products during sequencing library construction. More specifically, the present invention relates to a method for removing the linker self-linking products during the sequencing library construction by using an endonuclease, a method for constructing a sequencing library based on an RNA sample, a kit for constructing an RNA sequencing library, and a method for determining sequence information of an RNA molecule. The present invention further relates to a method and a related kit for simultaneous sequencing of DNA and RNA molecules in the same sample. More specifically, the present invention relates to a method for constructing a sequencing library based on a mixed system of DNA molecules and RNA molecules, a method for determining sequence information of the DNA and RNA molecules, and a method for determining sequence information of a DNA target region being transcribed and a synthesized RNA molecule in a transcription complex.

### Background Art

Small RNAs are a large class of regulatory molecules in organisms, including miRNA, siRNA, piRNA, snRNA, snoRNA, srRNA, etc. They play an important regulatory role in the organisms. In recent years, some studies have successfully used spectra of small RNAs as markers for diagnosing specific diseases. In the future, the detection of small RNAs will be widely used in early diagnosis, classification, and personalized detection and treatment of diseases. Commonly used small RNA quantitative detection technologies include deep sequencing technology, chip technology, and qRT-PCR technology. The latter two require the synthesis of specific probes and therefore can only detect known types of small RNAs. The high-throughput sequencing technology has been widely used in the small RNA research field for its advantages of a high throughput, high sensitivity, no need for any prior sequence information and secondary structure information, and ability to discover new small RNA molecules. The library construction method for deep sequencing of small RNAs is mainly a two-step linker ligation reverse transcription method. This method is applicable to construction of deep sequencing libraries of any RNA, including fragmented long transcript RNA, suitable for ligation reactions, such as small RNA sequencing, CLIP sequencing, RIP sequencing, GRO sequencing, etc.

Due to technical limitations of small RNA sequencing, it is still difficult for existing library construction methods of small RNAs or RNA fragments to detect small RNAs in micro samples (less than 100 ng total RNAs or 1 ng small RNAs). The steps for library construction of small RNAs or RNA fragments first require connecting a 3' linker sequence to the 3' end of a small RNA, and then connecting a 5' linker sequence to the 5' end of the small RNA or RNA fragment. After this part of products is reverse transcribed and PCR amplified, a library used for deep sequencing can be obtained. In a process of ligation reactions, the ligation reactions will occur between excess 5' linkers and 3' linkers, producing useless by-products. For ligation reactions of small RNAs or RNA fragments with very low starting amounts, the by-products produced by the ligation between the 5' and 3' linkers account for the vast majority, seriously hindering the subsequent PCR amplification of the library.

In the existing solutions, the CleanTag technology of Trilink adds special modifications at the termini of the 5' linkers and 3' linkers, and as a result, linker self-linking products cannot be effectively extended by a reverse transcriptase, thereby removing linker self-linking contamination, while Ligang Wu and others from the Shanghai Institutes for Biological Sciences, Chinese Academy of Sciences, remove the linker contamination by a method of using a Cas9 enzyme to cleave the linker self-linking products, and a corresponding sequencing method is CAS-seq. In these methods, the CleanTag technology is costly and can only partially remove the contamination, while the CAS-seq technology may have off-target effects and cleave small RNA inserted fragments due to the use of the Cas9 enzyme, and is costly and complicated in experimental operations.

Therefore, how to simply and efficiently remove the by-products produced by the ligation between the 5' and 3' linkers is the key to achieving library construction of trace amounts of small RNAs or RNA fragments.

Integrated analysis of DNA and RNA sequencing data is conducive to exploring genetic characteristics in normal phenotypes and diseases. In addition to being used to calculate gene expression levels, the RNA sequencing data can further be used for verifying base variation data on DNA in parallel. For example, in cancer, approximately one-third of somatic single nucleotide variants (SNVs) located in a coding region can also be observed in the RNA data, providing a biological filtering method for screening true variant sites. In addition, the integrated analysis of the DNA and RNA data is very useful for analyzing differences in gene expression regulation, RNA editing, and allele-specific expression, and is very important for analyzing disease samples.

Currently, most DNA and RNA integrated analysis experiments are to perform DNA and RNA sequencing on samples in parallel, and this strategy requires a long library preparation time and may cause an analytic deviation due to sample heterogeneity. Existing single-cell integrated sequencing methods G&T-seq and DR-seq have discovered the correlation between copy numbers and expression at the cellular level on a genome-wide scale. However, due to batch effects and coverage problems inherent in the current single-cell sequencing methods, these new methods are of limited effectiveness in situations where more comprehensive genomes and transcriptomes are required. In addition, these two methods still require constructing DNA and RNA sequencing libraries separately, and cannot accurately retain physical association information of DNA and RNA molecules in the samples. Simul-seq can simultaneously construct libraries for DNA and RNA samples extracted from tissue samples, but its steps rely on fragmentation and linker connections by a Tn5 enzyme on DNA, and on a characteristic that an RNA ligase 1 can only connect single strands to distinguish the RNA molecules. This method will have a 5' terminus deviation, and cannot distinguish single-stranded DNA and RNA molecules. In addition, because the DNA will be fragmented in a process in which the Tn5 adds linkers, Simul-seq is also not applicable to mixed samples of short-segment DNA and RNA molecules, such as cfDNA and cfRNA mixtures extracted from plasma, whereas such samples are of great significance for cancer screening, cancer auxiliary diagnosis, and postoperative follow-up.

Therefore, there is still a need to improve and invent a new method for simultaneously producing DNA and RNA sequencing data, so as to perform comprehensive genomic and transcriptomic analysis on samples such as plasma.

### Summary of the Invention

Specifically, the present invention solves the foregoing technical problems existing in the prior art through the following technical solutions.

Item 1. A method for constructing a sequencing library for an RNA molecule in a sample, comprising the steps of:
(1) connecting a 3' end linker to the 3' terminus of the RNA molecule in the sample;
(2) connecting a 5' end linker to the 5' terminus of the RNA molecule in the sample connected with the 3' end linker;
(3) obtaining an extension product using an elongase and an extension primer based on the RNA molecule in the sample connected with the 3' end linker and the 5' end linker;
(4) treating the extension product using a DNA endonuclease and an enzyme capable of specifically hydrolyzing an RNA in a DNA-RNA heterozygous chain; and
(5) performing PCR amplification on the extension product to obtain the sequencing library,
wherein both the 5' end linker and the 3' end linker contain a partial sequence of a recognition site of the DNA endonuclease, and when a self-linking product of the 5' end linker and the 3' end linker is formed, a complete sequence of the recognition site is formed at the junction; and preferably, the enzyme capable of specifically hydrolyzing the RNA in the DNA-RNA heterozygous chain is RNase H.

Item 2. The method according to Item 1, wherein the ligation reaction of step (1) uses a truncated T4 RNA ligase 2 or a point mutant thereof, and/or the ligation reaction of step (2) uses a T4 RNA ligase 1.

Item 3. The method according to Item 1 or 2, wherein the elongase used in step (3) is a reverse transcriptase and/or Bst polymerase; and preferably, the reverse transcriptase is an MMLV reverse transcriptase.

Item 4. The method according to any one of Items 1 to 3, wherein the RNA molecule in the sample is a small RNA molecule; and preferably, the small RNA molecule has a length of 15-200 nt.

Item 5. The method according to any one of Items 1 to 4, wherein a content of the RNA molecules in the sample is ≤ 100 pg, ≤ 200 pg, ≤ 500 pg, ≤ 1 ng, ≤ 2 ng, ≤ 5 ng, ≤ 10 ng, or ≤ 20 ng.

Item 6. The method according to any one of Items 1 to 5, wherein the content of the RNA molecules in the sample is ≥ 50 pg; and preferably, the content of the RNA molecules in the sample is 50 pg-20 ng.

Item 7. A method for processing a sample containing a DNA double strand and a DNA single strand, wherein the method comprises adding a DNA endonuclease to the sample, and
a DNA:DNA pairing region of the DNA double strand contains a sequence of a recognition site of the DNA endonuclease;
optionally, the DNA single strand and the DNA double strand are generated during construction of an RNA sequencing library;
optionally, the DNA double strand comprises a self-linking product formed by a 5' end linker and a 3' end linker used during RNA sequencing library construction; and
optionally, when the self-linking product is formed, the sequence of the recognition site of the DNA endonuclease is formed at the junction of the 5' end linker and the 3' end linker.

Item 8. The method according to any one of Items 1 to 7, wherein the method can efficiently remove the self-linking product formed by the 5' end linker and the 3' end linker in a system; and preferably, the method can remove at least 50%, at least 60%, at least 70%, at least 80%, at least 90%, at least 95%, at least 98%, at least 99%, at least 99.5%, at least 99.8%, or at least 99.9% of the self-linking products in the system.

Item 9. The method according to any one of Items 1 to 8, wherein the 5' end of the 3' end linker carries at least one, at least two, at least three, or at least four consecutive nucleotides of the 3' end of the recognition site of the DNA endonuclease; optionally, the 5' end of the 3' end linker has an adenylation modification, and the 3' end has a dideoxy modification; and optionally, the 3' end linker is a DNA linker.

Item 10. The method according to any one of Items 1 to 9, wherein the 3' end of the 5' end linker carries at least one, at least two, at least three, or at least four consecutive nucleotides of the 5' end of the recognition site of the DNA endonuclease; and optionally, the 5' end linker is a DNA linker.

Item 11. The method according to any one of Items 1 to 10, wherein neither the 5' end linker nor the 3' end linker comprises the complete sequence of the recognition site of the DNA endonuclease.

Item 12. The method according to any one of Items 1 to 11, wherein the DNA endonuclease can effectively cleave the DNA double strand containing the sequence of the recognition site thereof, but has low activity for a DNA single strand containing the sequence of the recognition site.

Item 13. The method according to Item 12, wherein a cleavage efficiency of the DNA endonuclease for the DNA single strand containing the sequence of the recognition site thereof is at most 1/10, at most 1/100, at most 1/1000, or at most 1/10000 of a cleavage efficiency for a corresponding DNA:DNA double strand, the DNA:DNA double strand contains the DNA single strand of the same sequence and a DNA single strand complementary thereto; and preferably, the DNA endonuclease is inactive, or has no detectable activity, for the DNA single strand containing the sequence of the recognition site thereof.

Item 14. The method according to any one of Items 1 to 13, wherein the DNA endonuclease is a double-stranded DNA endonuclease; and preferably, the DNA endonuclease is selected from the group of: AatII, BamHI, BsaBI, BsrFI, DraI, HphI, NdeI, Paul, PvuII, SwaI, Acc651, BanI, BsaHI, BsrGI, DraIII, Hpy188I, NgoMI, RsaI, TaqI, AccI, BanII, BsaI, BsrI, DrdI, Hpy188III, NheI, RsrII, TfiI, AciI, BbsI, BsaJi, BssHI, BssHII, EaeI, Hpy99I, NlaIII, SacI, TliI, AclI, BbvCI, BsaWI, BssKI, EagI, HpyCH4III, NlaIV, SacII, TseI, AcuI, BbvI, BsaXI, BssSI, EarI, HpyCH4IV, NotI, SalI, Tsp451, AfeI, BccI, BseRI, BstAPI, EciI, HpyCH4V, NruI, SapI, Tsp509I, AflII, BceAI, BseYI, BstBI, EcoNI, KasI, NsiI, Sau3AI, TspRI, AflIII, BcgI, BsgI, BstEII, EcoO109I, KpnI, NspI, Sau96I, Tth111I, AgeI, BciVI, BsiEI, BstF5I, EcoRI, MboI, PacI, Sbfl, XbaI, AhdI, Bell, BsiHKAI, BstNI, EcoRV, MboII, PaeR7I, Seal, XcmI, AleI, BfaI, BsiWI, BstUI, FatI, MfeI, PciI, ScrFI, XhoI, AluI, BfrBI, BsiI, BstXI, Paul, MluI, PflFI, SexAI, XmaI, AlwI, BfuAI, BsmAI, BstYI, Fnu4HI, MlyI, PflMI, SfaNI, XmnI, AlwNI, BfuCI, BsmBI, BstZ17I, FseI, MmeI, PhoI, SfcI, ZraI, ApaI, BglI, BsmFI, Bsu36I, FspI, MnlI, PleI, SfoI, ApaLI, BglII, BsmI, BtgI, HaeII, MscI, PmeI, SgrAI, Nb.BbvCI, ApeKI, BlpI, BsoBI, BtgZI, HaeIII, MseI, PmlI, SmaI, Nt.BbvCI, ApoI, Bme1580I, Bsp1286I, BtsI, HgaI, MsiI, PpuMI, SmlI, Nb.BsmI, AscI, BmgBI, BspCNI, Cac8I, HhaI, MspA1I, PshAI, SnaBI, Nt.BstNBI, AseI, BmrI, BspDI, ClaI, HincII, MspI, PsiI, SpeI, AsiSI, BmtI, BspEI, CspCI, HindIII, MwoI, PspGI, SphI, AvaI, BpmI, BspHI, CviAII, Hinfl, NaeI, PspOMI, SspI, AvaII, Bpu10I, BspMI, DdeI, HinP1I, NarI, PspXI, StuI, AvrII, BpuEI, BsrBI, DpnI, HpaI, NciI, PstI, StyD4I, BaeI, BsaAI, BsrDI, DpnII, HpaII, NcoI, PvuI, and StyI.

Item 15. A method for determining sequence information of a small RNA molecule, comprising:
constructing a sequencing library based on a small RNA molecule sample by the method according to any one of Items 1 to 14; sequencing the sequencing library to obtain a sequencing result; and determining the sequence information of the small RNA molecule based on the sequencing result.

Item 16. A kit for constructing an RNA sequencing library, comprising:
an RNA 3' terminus connecting module, comprising a 3' end linker of an RNA molecule; and
an RNA 5' terminus connecting module, comprising a 5' end linker of the RNA molecule;
wherein both the 5' end linker and the 3' end linker contain a partial sequence of a recognition site of a DNA endonuclease, and when a self-linking product of the 5' end linker and the 3' end linker is formed, a complete sequence of the recognition site is formed at the junction.

Item 17. The kit according to Item 16, wherein the 5' end of the 3' end linker carries at least one, at least two, at least three, or at least four consecutive nucleotides of the 3' end of the recognition site of the DNA endonuclease; optionally, the 5' end of the 3' end linker has an adenylation modification, and the 3' end has a dideoxy modification; and optionally, the 3' end linker is a DNA linker.

Item 18. The kit according to any one of Items 16 to 17, wherein the 3' end of the 5' end linker carries at least one, at least two, at least three, or at least four consecutive nucleotides of the 5' end of the recognition site of the DNA endonuclease; and optionally, the 5' end linker is a DNA linker.

Item 19. The kit according to any one of Items 16 to 18, wherein neither the 5' end linker nor the 3' end linker comprises the complete sequence of the recognition site of the DNA endonuclease.

Item 20. The kit according to any one of Items 16 to 19, wherein
(a) the RNA 3' terminus connecting module comprises a first ligase; and preferably, the first ligase is a truncated T4 RNA ligase 2 or a point mutant thereof; and/or
(b) the RNA 5' terminus connecting module comprises a second ligase, and preferably, the second ligase is a T4 RNA ligase 1.

Item 21. The kit according to any one of Items 16 to 20, further comprising an enzyme cleavage module, wherein the enzyme cleavage module comprises the DNA endonuclease; optionally, the enzyme cleavage module further comprises an enzyme capable of specifically hydrolyzing an RNA in a DNA-RNA heterozygous chain; and optionally, the enzyme capable of specifically hydrolyzing the RNA in the DNA-RNA heterozygous chain is RNase H.

Item 22. The kit according to any one of Items 16 to 21, further comprising an extension module, wherein the extension module comprises an elongase for extending an RNA molecule connected with the 3' end linker and the 5' end linker; and preferably, the extension module and the enzyme cleavage module can be integrated into one module.

Item 23. The kit according to Item 22, wherein the elongase comprises a reverse transcriptase and/or Bst polymerase; and preferably, the reverse transcriptase is an MMLV reverse transcriptase.

Item 24. The kit according to any one of Items 16 to 23, further comprising an amplification module, wherein the amplification module comprises an enzyme that is required for DNA amplification and can be used for amplifying a product of the enzyme cleavage module.

Item 25. The kit according to any one of Items 16 to 24, wherein the RNA is a small RNA; and preferably, the small RNA has a length of 15-200 nt.

Item 26. The kit according to any one of Items 16 to 25, wherein the DNA endonuclease can effectively cleave a DNA double strand containing the sequence of the recognition site thereof, but has low activity for a DNA single strand containing the sequence of the recognition site.

Item 27. The kit according to Item 26, wherein a cleavage efficiency of the DNA endonuclease for the DNA single strand containing the sequence of the recognition site thereof is at most 1/10, at most 1/100, at most 1/1000, or at most 1/10000 of a cleavage efficiency for a corresponding DNA:DNA double strand, the DNA:DNA double strand contains the DNA single strand of the same sequence and a DNA single strand complementary thereto; and preferably, the DNA endonuclease is inactive, or has no detectable activity, for the DNA single strand containing the sequence of the recognition site thereof.

Item 28. The kit according to any one of Items 16 to 27, wherein the DNA endonuclease is a double-stranded DNA endonuclease; and preferably, the DNA endonuclease is selected from the group of: AatII, BamHI, BsaBI, BsrFI, DraI, HphI, NdeI, Paul, PvuII, SwaI, Acc651, BanI, BsaHI, BsrGI, DraIII, Hpy188I, NgoMI, RsaI, TaqI, AccI, BanII, BsaI, BsrI, DrdI, Hpy188III, NheI, RsrII, TfiI, AciI, BbsI, BsaJi, BssHI, BssHII, EaeI, Hpy99I, NlaIII, SacI, TliI, AclI, BbvCI, BsaWI, BssKI, EagI, HpyCH4III, NlaIV, SacII, TseI, AcuI, BbvI, BsaXI, BssSI, EarI, HpyCH4IV, NotI, SalI, Tsp451, AfeI, BccI, BseRI, BstAPI, EciI, HpyCH4V, NruI, SapI, Tsp509I, AflII, BceAI, BseYI, BstBI, EcoNI, KasI, NsiI, Sau3AI, TspRI, AflIII, BcgI, BsgI, BstEII, EcoO109I, KpnI, NspI, Sau96I, Tth111I, AgeI, BciVI, BsiEI, BstF5I, EcoRI, MboI, PacI, Sbfl, XbaI, AhdI, Bell, BsiHKAI, BstNI, EcoRV, MboII, PaeR7I, ScaI, XcmI, AleI, BfaI, BsiWI, BstUI, FatI, MfeI, PciI, ScrFI, XhoI, AluI, BfrBI, BsiI, BstXI, Paul, MluI, PflFI, SexAI, XmaI, AlwI, BfuAI, BsmAI, BstYI, Fnu4HI, MlyI, PflMI, SfaNI, XmnI, AlwNI, BfuCI, BsmBI, BstZ17I, FseI, MmeI, PhoI, SfcI, ZraI, ApaI, BglI, BsmFI, Bsu36I, FspI, MnlI, PleI, SfoI, ApaLI, BglII, BsmI, BtgI, HaeII, MscI, PmeI, SgrAI, Nb.BbvCI, ApeKI, BlpI, BsoBI, BtgZI, HaeIII, MseI, PmlI, SmaI, Nt.BbvCI, ApoI, Bme1580I, Bsp1286I, BtsI, HgaI, MsiI, PpuMI, SmlI, Nb.BsmI, AscI, BmgBI, BspCNI, Cac8I, HhaI, MspA1I, PshAI, SnaBI, Nt.BstNBI, AseI, BmrI, BspDI, ClaI, HincII, MspI, PsiI, SpeI, AsiSI, BmtI, BspEI, CspCI, HindIII, MwoI, PspGI, SphI, AvaI, BpmI, BspHI, CviAII, Hinfl, NaeI, PspOMI, SspI, Avail, Bpu10I, BspMI, DdeI, HinP1I, NarI, PspXI, StuI, AvrII, BpuEI, BsrBI, DpnI, HpaI, NciI, PstI, StyD4I, BaeI, BsaAI, BsrDI, DpnII, HpaII, NcoI, PvuI, and StyI.

Item 29. A method for constructing a sequencing library for an RNA molecule in a sample, comprising the steps of:
(1) connecting a 3' end linker to the 3' terminus of the RNA molecule in the sample;
(2) connecting a 5' end linker to the 5' terminus of the RNA molecule in the sample connected with the 3' end linker;
(3) obtaining an extension product using an elongase and an extension primer based on the RNA molecule in the sample connected with the 3' end linker and the 5' end linker;
(4) treating the extension product using a DNA endonuclease; and
(5) performing PCR amplification on the extension product to obtain the sequencing library,
wherein both the 5' end linker and the 3' end linker contain a partial sequence of a recognition site of the DNA endonuclease, and when a self-linking product of the 5' end linker and the 3' end linker is formed, a complete sequence of the recognition site is formed at the junction.

Item 30. The method according to Item 29, wherein the ligation reaction of step (1) uses a truncated T4 RNA ligase 2 or a point mutant thereof, and/or the ligation reaction of step (2) uses a T4 RNA ligase 1.

Item 31. The method according to Item 29 or 30, wherein the elongase used in step (3) is a reverse transcriptase and/or Taq enzyme; and preferably, the reverse transcriptase is an MMLV reverse transcriptase.

Item 32. The method according to any one of Items 29 to 31, wherein the RNA molecule in the sample is a small RNA molecule; and preferably, the small RNA molecule has a length of 15-200 nt.

Item 33. The method according to any one of Items 29 to 32, wherein a content of the RNA molecules in the sample is ≤ 100 pg, ≤ 200 pg, ≤ 500 pg, ≤ 1 ng, ≤ 2 ng, ≤ 5 ng, ≤ 10 ng, or ≤ 20 ng.

Item 34. The method according to any one of Items 29 to 33, wherein the content of the RNA molecules in the sample is ≥ 50 pg; and preferably, the content of the RNA molecules in the sample is 50 pg-20 ng.

Item 35. A method for processing a sample containing a DNA-RNA-DNA:cDNA heterozygous chain and a DNA:cDNA double strand, wherein the method comprises adding a DNA endonuclease to the sample, and
a DNA:cDNA pairing region of the DNA:cDNA double strand contains a sequence of a recognition site of the DNA endonuclease;
optionally, an RNA:cDNA pairing region of the DNA-RNA-DNA:cDNA heterozygous chain contains the sequence of the recognition site;
optionally, the DNA-RNA-DNA:cDNA heterozygous chain is generated during construction of an RNA sequencing library;
optionally, the DNA:cDNA double strand comprises a self-linking product formed by a 5' end linker and a 3' end linker used during RNA sequencing library construction; and
optionally, when the self-linking product is formed, the sequence of the recognition site of the DNA endonuclease is formed at the junction of the 5' end linker and the 3' end linker.

Item 36. The method according to any one of Items 29 to 35, wherein the method can efficiently remove the self-linking product formed by the 5' end linker and the 3' end linker in a system; and preferably, the method can remove at least 50%, at least 60%, at least 70%, at least 80%, at least 90%, at least 95%, at least 98%, at least 99%, at least 99.5%, at least 99.8%, or at least 99.9% of the self-linking products in the system.

Item 37. The method according to any one of Items 29 to 36, wherein the 5' end of the 3' end linker carries at least one, at least two, at least three, or at least four consecutive nucleotides of the 3' end of the recognition site of the DNA endonuclease; optionally, the 5' end of the 3' end linker has an adenylation modification, and the 3' end has a dideoxy modification; and optionally, the 3' end linker is a DNA linker.

Item 38. The method according to any one of Items 29 to 37, wherein the 3' end of the 5' end linker carries at least one, at least two, at least three, or at least four consecutive nucleotides of the 5' end of the recognition site of the DNA endonuclease; and optionally, the 5' end linker is a DNA linker.

Item 39. The method according to any one of Items 29 to 38, wherein neither the 5' end linker nor the 3' end linker comprises the complete sequence of the recognition site of the DNA endonuclease.

Item 40. The method according to any one of Items 29 to 39, wherein the DNA endonuclease can effectively cleave a DNA double strand containing the sequence of the recognition site thereof, but has low activity for an RNA:DNA heterozygous chain containing the sequence of the recognition site.

Item 41. The method according to Item 40, wherein a cleavage efficiency of the DNA endonuclease for an RNA:cDNA heterozygous chain containing the sequence of the recognition site thereof is at most 1/10, at most 1/100, at most 1/1000, or at most 1/10000 of a cleavage efficiency for the corresponding DNA:cDNA double strand, and the DNA:cDNA double strand contains a cDNA of the same sequence and a DNA complementary thereto; and preferably, the DNA endonuclease is inactive, or has no detectable activity, for the RNA:cDNA heterozygous chain containing the sequence of the recognition site thereof.

Item 42. The method according to any one of Items 29 to 41, wherein the DNA endonuclease is a double-stranded DNA endonuclease; and preferably, the DNA endonuclease is selected from the group of: AatII, BamHI, BsaBI, BsrFI, DraI, HphI, NdeI, PauI, PvuII, SwaI, Acc651, BanI, BsaHI, BsrGI, DraIII, Hpy188I, NgoMI, RsaI, TaqI, AccI, BanII, BsaI, BsrI, DrdI, Hpy188III, NheI, RsrII, TfiI, AciI, BbsI, BsaJi, BssHI, BssHII, EaeI, Hpy99I, NlaIII, SacI, TliI, AclI, BbvCI, BsaWI, BssKI, EagI, HpyCH4III, NlaIV, SacII, TseI, AcuI, BbvI, BsaXI, BssSI, EarI, HpyCH4IV, NotI, SalI, Tsp451, AfeI, BccI, BseRI, BstAPI, EciI, HpyCH4V, NruI, SapI, Tsp509I, AflII, BceAI, BseYI, BstBI, EcoNI, KasI, NsiI, Sau3AI, TspRI, AflIII, BcgI, BsgI, BstEII, EcoO109I, KpnI, NspI, Sau96I, Tth111I, AgeI, BciVI, BsiEI, BstF5I, EcoRI, MboI, PacI, Sbfl, Xbal, AhdI, Bell, BsiHKAI, BstNI, EcoRV, MboII, PaeR7I, Seal, XcmI, AleI, BfaI, BsiWI, BstUI, FatI, MfeI, PciI, ScrFI, XhoI, AluI, BfrBI, BsiI, BstXI, Paul, MluI, PflFI, SexAI, XmaI, AlwI, BfuAI, BsmAI, BstYI, Fnu4HI, MlyI, PflMI, SfaNI, XmnI, AlwNI, BfuCI, BsmBI, BstZ17I, FseI, MmeI, PhoI, SfcI, ZraI, ApaI, BglI, BsmFI, Bsu36I, FspI, MnlI, PleI, SfoI, ApaLI, BglII, BsmI, BtgI, HaeII, MscI, PmeI, SgrAI, Nb.BbvCI, ApeKI, BlpI, BsoBI, BtgZI, HaeIII, MseI, PmlI, SmaI, Nt.BbvCI, ApoI, Bme1580I, Bsp1286I, BtsI, HgaI, MsiI, PpuMI, SmlI, Nb.BsmI, AscI, BmgBI, BspCNI, Cac8I, HhaI, MspA1I, PshAI, SnaBI, Nt.BstNBI, AseI, BmrI, BspDI, ClaI, HincII, MspI, PsiI, SpeI, AsiSI, BmtI, BspEI, CspCI, HindIII, MwoI, PspGI, SphI, AvaI, BpmI, BspHI, CviAII, Hinfl, NaeI, PspOMI, SspI, AvaII, Bpu10I, BspMI, DdeI, HinP1I, NarI, PspXI, StuI, AvrII, BpuEI, BsrBI, DpnI, HpaI, NciI, PstI, StyD4I, BaeI, BsaAI, BsrDI, DpnII, HpaII, Ncol, PvuI, and StyI; and preferably, the double-stranded DNA endonuclease is not AvaII, AvrII, BanI, HaeIII, Hinfl, TaqI, or other enzymes that can cleave a DNA/RNA heterozygous chain under specific conditions.

Item 43. A method for determining sequence information of a small RNA molecule, comprising:
constructing a sequencing library based on a small RNA molecule sample by the method according to any one of Items 29 to 42; sequencing the sequencing library to obtain a sequencing result; and determining the sequence information of the small RNA molecule based on the sequencing result.

Item 44. The kit according to any one of Items 22 to 28, wherein the elongase comprises a reverse transcriptase and/or Taq enzyme; and preferably, the reverse transcriptase is an MMLV reverse transcriptase.

Item 45. The kit according to any one of Items 16 to 28 and 44, wherein the DNA endonuclease can effectively cleave the DNA double strand containing the sequence of the recognition site thereof, but has low activity for an RNA:DNA heterozygous chain containing the sequence of the recognition site.

Item 46. The kit according to Item 45, wherein a cleavage efficiency of the DNA endonuclease for an RNA:cDNA heterozygous chain containing the sequence of the recognition site thereof is at most 1/10, at most 1/100, at most 1/1000, or at most 1/10000 of a cleavage efficiency for the corresponding DNA:cDNA double strand, and the DNA:cDNA double strand contains a cDNA of the same sequence and a DNA complementary thereto; and preferably, the DNA endonuclease is inactive, or has no detectable activity, for the RNA:cDNA heterozygous chain containing the sequence of the recognition site thereof.

Item 47. The kit according to any one of Items 16 to 28 and 44 to 46, wherein the double-stranded DNA endonuclease is not Avail, AvrII, BanI, HaeIII, Hinfl, TaqI, or other enzymes that can cleave a DNA/RNA heterozygous chain under specific conditions.

Item 48. A system for determining sequence information of an RNA molecule, comprising:
the kit according to any one of Items 16 to 28 and 44 to 47;
a sequencing device, used for sequencing the sequencing library constructed for a sample by the kit to obtain a sequencing result of the sample; and
an analysis device, for analyzing the sequencing result to determine the sequence information of the RNA molecule; wherein
preferably, the RNA molecule is a small RNA molecule.

Item 49. Use of the method according to any one of Items 1 to 15 and 29 to 43, or the kit according to any one of Items 16 to 28 and 44 to 47, or the system according to Item 48 in construction of an RNA sequencing library.

Item 50. The use according to Item 49, wherein the RNA sequencing library is selected from the group of: a plasma small RNA sequencing library, a CLIP library, an RIP library, an MeRIP library, and a GRO library.

Item 51. A method for constructing a sequencing library, comprising the steps of:
(1) connecting a specific tag linker to the 3' terminus of an RNA molecule in a mixed sample of DNA and RNA molecules;
(2) obtaining a single-stranded DNA molecule using a reverse transcription primer based on the RNA molecule connected with the tag linker; and
(3) constructing a library for all DNA molecules in the sample.

Item 52. A method for simultaneous sequencing of DNA and RNA molecules in a sample, comprising the steps of:
(1) connecting a specific tag linker to the 3' terminus of an RNA molecule in a mixed sample of DNA and RNA molecules;
(2) obtaining a single-stranded DNA molecule using a reverse transcription primer based on the RNA molecule connected with the tag linker;
(3) constructing a library for all DNA molecules in the sample; and
(4) sequencing the library, and using a sequence of the specific tag linker to distinguish the RNA molecules in the mixed sample.

Item 53. The method according to Item 51 or 52, wherein a ligase in the ligation reaction of step (1) is a truncated T4 RNA ligase 2 or a point mutant thereof.

Item 54. The method according to any one of Items 51 to 53, wherein a library construction method used in step (3) comprises:
(a) denaturing the DNA molecule into a single strand;
(b) connecting specific linkers to the 3' terminus and/or 5' terminus of the single strand obtained in step (a); and
(c) optionally, using an amplification primer for amplification to obtain an amplification product.

Item 55. The method according to any one of Items 51 to 53, wherein a library construction method used in step (3) comprises:
(A) extending the single-stranded DNA molecule obtained in step (2) into a double-stranded DNA;
(B) connecting double-stranded linkers to one or both ends of the double-stranded DNA obtained in step (A); and
(C) optionally, using an amplification primer for amplification to obtain an amplification product.

Item 56. The method according to Item 54, wherein the library construction method used in step (3) further comprises:
(d) extending the product obtained in step (b) into a double-stranded DNA;
(e) connecting double-stranded linkers to one or both ends of the double-stranded DNA obtained in step (d); and
(f) optionally, using an amplification primer for amplification to obtain an amplification product.

Item 57. The method according to any one of Items 52 to 56, wherein a sequencing method used in step (4) comprises using next-generation high-throughput sequencing or third-generation sequencing.

Item 58. The method according to any one of Items 51 to 57, wherein the 5' end of the specific tag linker has an adenylation modification, and the 3' end has a dideoxy modification; and optionally, the specific tag linker is a DNA linker.

Item 59. The method according to any one of Items 51 to 58, wherein the ligase used in the ligation reaction in step (1) can connect the specific linker to the 3' terminus of the RNA molecule but cannot connect the specific linker to the 3' terminus of the DNA molecule.

Item 60. A method for determining sequence information of DNA and RNA molecules in a mixed sample of the DNA and RNA molecules, comprising:
based on the mixed sample of the DNA and RNA molecules, constructing a sequencing library by the method according to any one of Items 51 to 57 and performing sequencing to obtain a sequencing result; and
based on the sequencing result, determining the sequence information of the DNA and RNA molecules in the mixed sample of the DNA and RNA molecules.

Item 61. A method for determining sequence information of a DNA target region being transcribed and a synthesized RNA molecule in a transcription complex, comprising:
(i) obtaining a DNA and RNA sample of the region being transcribed;
(ii) constructing a sequencing library for the sample by the method according to any one of Items 51 to 57 and performing sequencing to obtain a sequencing result;
(iii) based on the sequencing result, determining the sequence information of the DNA target region being transcribed and the synthesized RNA molecule.

Item 62. The method according to Item 61, wherein step (i) comprises:
randomly fragmenting chromatin in the sample, and performing co-immunoprecipitation on the chromatin using an antibody for an RNA polymerase, to obtain the DNA and RNA sample of the region being transcribed.

Item 63. The method according to Item 62, wherein the randomly fragmented chromatin has an average length of 200 bp-500 bp.

Item 64. The method according to Item 62 or 63, wherein step (i) comprises degrading proteins using proteinase K after the co-immunoprecipitation.

Item 65. The method according to any one of Items 51 to 64, wherein the sample contains a cell-free DNA and cell-free RNA.

Item 66. A kit for constructing a sequencing library of DNA and RNA molecules in the same sample, comprising:
an RNA terminus connecting module, which comprises a specific tag linker and is used for connecting the tag linker to the 3' terminus of the RNA molecule.

Item 67. The kit according to Item 66, further comprising:
a reverse transcription module, which comprises a reverse transcription primer and a reverse transcriptase and is used for generating a DNA molecule based on the RNA molecule connected with the tag linker.

Item 68. The kit according to Item 66 or 67, further comprising:
a DNA library construction module, which is used for constructing the DNA molecule in the sample into the sequencing library.

Item 69. The kit according to any one of Items 66 to 68, wherein the RNA terminus connecting module comprises a ligase; and preferably, the ligase is a truncated T4 RNA ligase 2 or a point mutant thereof.

Item 70. The kit according to any one of Items 51 to 69, wherein the 5' end of the specific tag linker has an adenylation modification, and the 3' end has a dideoxy modification; and optionally, the specific tag linker is a DNA linker.

Item 71. A system for determining sequence information of DNA and RNA molecules, comprising:
the kit according to any one of Items 66 to 70;
a sequencing device, used for sequencing the sequencing library constructed for a sample by the kit to obtain a sequencing result of the sample; and
an analysis device, for analyzing the sequencing result of the sample to obtain the sequence information of the DNA and RNA molecules.

Item 72. Use of the method according to any one of Items 51 to 65, or the kit according to any one of Items 66 to 70, or the system according to Item 71 in simultaneous sequencing of DNA and RNA molecules in the same sample.

### Brief Description of the Drawings

The foregoing and/or additional aspects and advantages of the present invention will become apparent and easily understood from the description of the embodiments with reference to the following drawings, wherein:
FIG. 1 shows a schematic flow chart of a method for removing linker self-linking products during sequencing library construction and a method for constructing a small RNA library according to one embodiment of the present invention;
FIG. 2 shows a quality inspection chart of fragment sizes of a small RNA library constructed according to one embodiment of the present invention; wherein a self-linking length of a linker is 144 nt, and an input amount is 0.5 ng small RNAs;
FIG. 3 shows a schematic flow chart of a method for removing linker self-linking products during sequencing library construction and a method for constructing a small RNA library according to one embodiment of the present invention;
FIG. 4 shows a quality inspection chart of fragment sizes of a small RNA library constructed according to one embodiment of the present invention; wherein a self-linking length of a linker is 144 nt, and an input amount is 0.5 ng small RNAs;
FIG. 5 shows a quality inspection chart of fragment sizes of a small RNA library constructed according to one embodiment of the present invention; wherein the upper band in the chart is a small RNA library using the present embodiment, and the lower band is a small RNA library using the same process as the present embodiment, but with a step of endonuclease treatment removed;
FIG. 6 shows a schematic flow chart of a method for simultaneous sequencing of DNA and RNA molecules in the same sample according to one embodiment of the present invention; and
FIG. 7 shows a quality inspection chart of fragment sizes of a DNA/RNA mixed library constructed according to one embodiment of the present invention.

### Detailed Description of Embodiments

The present invention provides the following methods and kits that can be used for sequencing library construction and sequencing:
1) ***Using DNA single and double strands to remove linker self-linking products:*** A method and a corresponding kit for selective cleavage of DNA double strands and DNA single strands are provided. The method comprises a step of mixing an DNA endonuclease with the DNA double strand and DNA single strand: wherein an enzyme cleavage site in the DNA double strand is located in a DNA:DNA pairing region; the DNA endonuclease can recognize the enzyme cleavage site in the DNA double strand and perform cleavage; optionally, the DNA double strand and DNA single strand are generated during construction of an RNA sequencing library; and optionally, the DNA double strand is a self-linking product formed by a 5' linker and a 3' linker used during RNA sequencing library construction.
2) ***Using DNA double strands and RNA:DNA heterozygous chains to remove linker self-linking products:*** A method and a corresponding kit for selective cleavage using DNA-RNA-DNA:cDNA heterozygous chains and DNA:cDNA double strands are provided. The method comprises a step of mixing an DNA endonuclease with the DNA-RNA-DNA:cDNA heterozygous chain and DNA:cDNA double strand: wherein an enzyme cleavage site in the DNA-RNA-DNA:cDNA heterozygous chain is located in an RNA:cDNA pairing region, an enzyme cleavage site in the DNA:cDNA double strand is located in a DNA:cDNA pairing region; the DNA endonuclease can recognize the enzyme cleavage site in the DNA:cDNA double strand and perform cleavage; optionally, the DNA-RNA-DNA:cDNA heterozygous chain is generated during construction of an RNA sequencing library; and optionally, the DNA is the 5' linker and 3' linker used during RNA sequencing library construction.
3) ***DNA and RNA library co-construction:*** A method for simultaneous sequencing of DNA and RNA molecules in the same sample is provided, wherein the sequencing library construction method comprises: (1) specifically connecting a tag linker to the 3' terminus of the RNA molecule; (2) obtaining a single-stranded DNA molecule using a reverse transcription primer based on the RNA molecule with the tag linker; and (3) constructing a library for all DNA molecules in a system. The method can effectively construct sequencing libraries of the DNA molecules and RNA molecules simultaneously, especially a sequencing library of a cfDNA/cfRNA (cell-free DNA/cell-free RNA).

According to one aspect of the present invention, a method for constructing a sequencing library for an RNA molecule in a sample is provided, comprising the steps of:
(1) connecting a 3' end linker to the 3' terminus of the RNA molecule in the sample;
(2) connecting a 5' end linker to the 5' terminus of the RNA molecule in the sample connected with the 3' end linker;
(3) obtaining an extension product using an elongase and an extension primer based on the RNA molecule in the sample connected with the 3' end linker and the 5' end linker;
(4) treating the extension product using a DNA endonuclease and an enzyme capable of specifically hydrolyzing an RNA in a DNA-RNA heterozygous chain; and
(5) performing PCR amplification on the extension product, to obtain the sequencing library,
wherein both the 5' end linker and the 3' end linker contain a partial sequence of a recognition site of the DNA endonuclease, and when a self-linking product of the 5' end linker and the 3' end linker is formed, a complete sequence of the recognition site is formed at the junction.

In some implementations, the enzyme capable of specifically hydrolyzing the RNA in the DNA-RNA heterozygous chain is RNase H.

According to one aspect of the present invention, a method for processing a sample containing a DNA double strand and a DNA single strand is provided, the method comprises adding a DNA endonuclease to the sample, and a DNA:DNA pairing region of the DNA double strand contains a sequence of a recognition site of the DNA endonuclease. In some implementations, the DNA single strand and the DNA double strand are generated during construction of an RNA sequencing library. In some implementations, the DNA double strand comprises a self-linking product formed by a 5' end linker and a 3' end linker used during RNA sequencing library construction. In some implementations, when the self-linking product is formed, the sequence of the recognition site of the DNA endonuclease is formed at the junction of the 5' end linker and the 3' end linker.

In some implementations, the ligation reaction of step (1) uses a truncated T4 RNA ligase 2 or a point mutant thereof, and/or the ligation reaction of step (2) uses a T4 RNA ligase 1.

In some implementations, the elongase used in step (3) is a reverse transcriptase and/or Bst polymerase. In some implementations, the reverse transcriptase is an MMLV reverse transcriptase.

In some implementations, the RNA molecule in the sample is a small RNA molecule. In some implementations, the small RNA molecule has a length of 15-200 nt.

In some implementations, a content of the RNA molecules in the sample is ≤ 100 pg, ≤ 200 pg, ≤ 500 pg, ≤ 1 ng, ≤ 2 ng, ≤ 5 ng, ≤ 10 ng, or ≤ 20 ng. In some implementations, the content of the RNA molecule in the sample is ≥ 50 pg. In some implementations, the content of the RNA molecule in the sample is 50 pg-20 ng.

In some implementations, the method can efficiently remove the self-linking product formed by the 5' end linker and the 3' end linker in a system. In some implementations, the method can remove at least 50%, at least 60%, at least 70%, at least 80%, at least 90%, at least 95%, at least 98%, at least 99%, at least 99.5%, at least 99.8%, or at least 99.9% of the self-linking products in the system.

In some implementations, the 5' end of the 3' end linker carries at least one, at least two, at least three, or at least four consecutive nucleotides of the 3' end of the recognition site of the DNA endonuclease. In some implementations, the 5' end of the 3' end linker has an adenylation modification. In some implementations, the 3' end of the 3' end linker has a dideoxy modification. In some implementations, the 3' end linker is a DNA linker.

In some implementations, the 3' end of the 5' end linker carries at least one, at least two, at least three, or at least four consecutive nucleotides of the 5' end of the recognition site of the DNA endonuclease. In some implementations, the 5' end linker is a DNA linker.

In some implementations, neither the 5' end linker nor the 3' end linker comprises a complete sequence of the recognition site of the DNA endonuclease.

In some implementations, the DNA endonuclease can effectively cleave the DNA double strand containing the sequence of the recognition site thereof, but has low activity for a DNA single strand containing the sequence of the recognition site. In some implementations, a cleavage efficiency of the DNA endonuclease for the DNA single strand containing the sequence of the recognition site thereof is at most 1/10, at most 1/100, at most 1/1000, or at most 1/10000 of a cleavage efficiency for a corresponding DNA:DNA double strand, the DNA:DNA double strand contains the DNA single strand of the same sequence and a DNA single strand complementary thereto. In some implementations, the DNA endonuclease is inactive, or has no detectable activity, for the DNA single strand containing the sequence of the recognition site thereof.

In some implementations, the DNA endonuclease is a double-stranded DNA endonuclease. In some implementations, the DNA endonuclease is selected from the group of: AatII, BamHI, BsaBI, BsrFI, DraI, HphI, NdeI, PauI, PvuII, SwaI, Acc651, BanI, BsaHI, BsrGI, DraIII, Hpy188I, NgoMI, RsaI, TaqI, AccI, BanII, BsaI, BsrI, DrdI, Hpy188III, NheI, RsrII, TfiI, AciI, BbsI, BsaJi, BssHI, BssHII, EaeI, Hpy99I, NlaIII, SacI, TliI, AclI, BbvCI, BsaWI, BssKI, EagI, HpyCH4III, NlaIV, SacII, TseI, AcuI, BbvI, BsaXI, BssSI, EarI, HpyCH4IV, NotI, SalI, Tsp451, AfeI, BccI, BseRI, BstAPI, EciI, HpyCH4V, NruI, SapI, Tsp509I, AflII, BceAI, BseYI, BstBI, EcoNI, KasI, NsiI, Sau3AI, TspRI, AflIII, BcgI, BsgI, BstEII, EcoO109I, KpnI, NspI, Sau96I, Tth111I, AgeI, BciVI, BsiEI, BstF5I, EcoRI, MboI, PacI, Sbfl, Xbal, AhdI, Bell, BsiHKAI, BstNI, EcoRV, MboII, PaeR7I, Seal, XcmI, AleI, BfaI, BsiWI, BstUI, FatI, MfeI, PciI, ScrFI, XhoI, AluI, BfrBI, BsiI, BstXI, Paul, MluI, PflFI, SexAI, XmaI, AlwI, BfuAI, BsmAI, BstYI, Fnu4HI, MlyI, PflMI, SfaNI, XmnI, AlwNI, BfuCI, BsmBI, BstZ17I, FseI, MmeI, PhoI, SfcI, ZraI, ApaI, BglI, BsmFI, Bsu36I, FspI, MnlI, PleI, SfoI, ApaLI, BglII, BsmI, BtgI, HaeII, MscI, PmeI, SgrAI, Nb.BbvCI, ApeKI, BlpI, BsoBI, BtgZI, HaeIII, MseI, PmlI, SmaI, Nt.BbvCI, ApoI, Bme1580I, Bsp1286I, BtsI, HgaI, MsiI, PpuMI, SmlI, Nb.BsmI, AscI, BmgBI, BspCNI, Cac8I, HhaI, MspA1I, PshAI, SnaBI, Nt.BstNBI, AseI, BmrI, BspDI, ClaI, HincII, MspI, PsiI, SpeI, AsiSI, BmtI, BspEI, CspCI, HindIII, MwoI, PspGI, SphI, AvaI, BpmI, BspHI, CviAII, Hinfl, NaeI, PspOMI, SspI, Avail, Bpu10I, BspMI, DdeI, HinP1I, NarI, PspXI, StuI, AvrII, BpuEI, BsrBI, DpnI, HpaI, NciI, PstI, StyD4I, BaeI, BsaAI, BsrDI, DpnII, HpaII, Ncol, PvuI, and StyI.

According to one aspect of the present invention, a method for determining sequence information of a small RNA molecule is provided, comprising constructing a sequencing library based on a small RNA molecule sample by the method described herein; sequencing the sequencing library to obtain a sequencing result; and determining the sequence information of the small RNA molecule based on the sequencing result.

According to one aspect of the present invention, a kit for constructing an RNA sequencing library is provided, comprising:
an RNA 3' terminus connecting module, comprising a 3' end linker of an RNA molecule; and
an RNA 5' terminus connecting module, comprising a 5' end linker of the RNA molecule;
wherein both the 5' end linker and the 3' end linker contain a partial sequence of a recognition site of a DNA endonuclease, and when a self-linking product of the 5' end linker and the 3' end linker is formed, a complete sequence of the recognition site is formed at the junction.

In some implementations, the 5' end of the 3' end linker carries at least one, at least two, at least three, or at least four consecutive nucleotides of the 3' end of the recognition site of the DNA endonuclease. In some implementations, the 5' end of the 3' end linker has an adenylation modification. In some implementations, the 3' end of the 3' end linker has a dideoxy modification. In some implementations, the 3' end linker is a DNA linker.

In some implementations, the 3' end of the 5' end linker carries at least one, at least two, at least three, or at least four consecutive nucleotides of the 5' end of the recognition site of the DNA endonuclease. In some implementations, the 5' end linker is a DNA linker.

In some implementations, neither the 5' end linker nor the 3' end linker comprises a complete sequence of the recognition site of the DNA endonuclease.

In some implementations, the RNA 3' terminus connecting module comprises a first ligase. In some implementations, the first ligase is a truncated T4 RNA ligase 2 or a point mutant thereof.

In some implementations, the RNA 5' terminus connecting module comprises a second ligase. In some implementations, the second ligase is a T4 RNA ligase 1.

In some implementations, the kit further comprises an enzyme cleavage module, and the enzyme cleavage module comprises the DNA endonuclease. In some implementations, the enzyme cleavage module further comprises an enzyme capable of specifically hydrolyzing an RNA in a DNA-RNA heterozygous chain. In some implementations, the enzyme capable of specifically hydrolyzing the RNA in the DNA-RNA heterozygous chain is RNase H.

In some implementations, the kit further comprises an extension module, and the extension module comprises an elongase for extending an RNA molecule connected with the 3' end linker and the 5' end linker. In some implementations, the extension module and the enzyme cleavage module can be integrated into one module.

In some implementations, the elongase comprises a reverse transcriptase and/or Bst polymerase. In some implementations, the reverse transcriptase is an MMLV reverse transcriptase.

In some implementations, the kit further comprises an amplification module, and the amplification module comprises an enzyme that is required for DNA amplification and can be used for amplifying a product of the enzyme cleavage module.

In some implementations, the RNA is a small RNA. In some implementations, the small RNA has a length of 15-200 nt.

In some implementations, the DNA endonuclease can effectively cleave the DNA double strand containing the sequence of the recognition site thereof, but has low activity for a DNA single strand containing the sequence of the recognition site. In some implementations, a cleavage efficiency of the DNA endonuclease for the DNA single strand containing the sequence of the recognition site thereof is at most 1/10, at most 1/100, at most 1/1000, or at most 1/10000 of a cleavage efficiency for a corresponding DNA:DNA double strand, the DNA:DNA double strand contains the DNA single strand of the same sequence and a DNA single strand complementary thereto. In some implementations, the DNA endonuclease is inactive, or has no detectable activity, for the DNA single strand containing the sequence of the recognition site thereof.

In some implementations, the DNA endonuclease is a double-stranded DNA endonuclease. In some implementations, the DNA endonuclease is selected from the group of: AatII, BamHI, BsaBI, BsrFI, DraI, HphI, NdeI, PauI, PvuII, SwaI, Acc651, BanI, BsaHI, BsrGI, DraIII, Hpy188I, NgoMI, RsaI, TaqI, AccI, BanII, BsaI, BsrI, DrdI, Hpy188III, NheI, RsrII, TfiI, AciI, BbsI, BsaJi, BssHI, BssHII, EaeI, Hpy99I, NlaIII, SacI, TliI, AclI, BbvCI, BsaWI, BssKI, EagI, HpyCH4III, NlaIV, SacII, TseI, AcuI, BbvI, BsaXI, BssSI, EarI, HpyCH4IV, NotI, SalI, Tsp451, AfeI, BccI, BseRI, BstAPI, EciI, HpyCH4V, NruI, SapI, Tsp509I, AflII, BceAI, BseYI, BstBI, EcoNI, KasI, NsiI, Sau3AI, TspRI, AflIII, BcgI, BsgI, BstEII, EcoO109I, KpnI, NspI, Sau96I, Tth111I, AgeI, BciVI, BsiEI, BstF5I, EcoRI, MboI, PacI, Sbfl, Xbal, AhdI, Bell, BsiHKAI, BstNI, EcoRV, MboII, PaeR7I, Seal, XcmI, AleI, BfaI, BsiWI, BstUI, FatI, MfeI, PciI, ScrFI, XhoI, AluI, BfrBI, BsiI, BstXI, Paul, MluI, PflFI, SexAI, XmaI, AlwI, BfuAI, BsmAI, BstYI, Fnu4HI, MlyI, PflMI, SfaNI, XmnI, AlwNI, BfuCI, BsmBI, BstZ17I, FseI, MmeI, PhoI, SfcI, ZraI, ApaI, BglI, BsmFI, Bsu36I, FspI, MnlI, PleI, SfoI, ApaLI, BglII, BsmI, BtgI, HaeII, MscI, PmeI, SgrAI, Nb.BbvCI, ApeKI, BlpI, BsoBI, BtgZI, HaeIII, MseI, PmlI, SmaI, Nt.BbvCI, ApoI, Bme1580I, Bsp1286I, BtsI, HgaI, MsiI, PpuMI, SmlI, Nb.BsmI, AscI, BmgBI, BspCNI, Cac8I, HhaI, MspA1I, PshAI, SnaBI, Nt.BstNBI, AseI, BmrI, BspDI, ClaI, HincII, MspI, PsiI, SpeI, AsiSI, BmtI, BspEI, CspCI, HindIII, MwoI, PspGI, SphI, AvaI, BpmI, BspHI, CviAII, Hinfl, NaeI, PspOMI, SspI, Avail, Bpu10I, BspMI, DdeI, HinP1I, NarI, PspXI, StuI, AvrII, BpuEI, BsrBI, DpnI, HpaI, NciI, PstI, StyD4I, BaeI, BsaAI, BsrDI, DpnII, HpaII, Ncol, PvuI, and StyI.

In some implementations, the elongase comprises a reverse transcriptase and/or Taq enzyme. In some implementations, the reverse transcriptase is an MMLV reverse transcriptase.

In some implementations, the DNA endonuclease can effectively cleave a DNA double strand containing the sequence of the recognition site thereof, but has low activity for an RNA:DNA heterozygous chain containing the sequence of the recognition site.

In some implementations, a cleavage efficiency of the DNA endonuclease for an RNA:cDNA heterozygous chain containing the sequence of the recognition site thereof is at most 1/10, at most 1/100, at most 1/1000, or at most 1/10000 of a cleavage efficiency for the corresponding DNA:cDNA double strand, and the DNA:cDNA double strand contains a cDNA of the same sequence and a DNA complementary thereto. In some implementations, the DNA endonuclease is inactive, or has no detectable activity, for the RNA:cDNA heterozygous chain containing the sequence of the recognition site thereof. In some implementations, the double-stranded DNA endonuclease is not Avail, AvrII, BanI, HaeIII, Hinfl, TaqI, or other enzymes that can cleave a DNA/RNA heterozygous chain under specific conditions.

According to one aspect of the present invention, a method for constructing a sequencing library for an RNA molecule in a sample is provided, comprising the steps of:
(1) connecting a 3' end linker to the 3' terminus of the RNA molecule in the sample;
(2) connecting a 5' end linker to the 5' terminus of the RNA molecule in the sample connected with the 3' end linker;
(3) obtaining an extension product using an elongase and an extension primer based on the RNA molecule in the sample connected with the 3' end linker and the 5' end linker;
(4) treating the extension product using a DNA endonuclease; and
(5) performing PCR amplification on the extension product, to obtain the sequencing library,
wherein both the 5' end linker and the 3' end linker contain a partial sequence of a recognition site of the DNA endonuclease, and when a self-linking product of the 5' end linker and the 3' end linker is formed, a complete sequence of the recognition site is formed at the junction.

In some implementations, the ligation reaction of step (1) uses a truncated T4 RNA ligase 2 or a point mutant thereof. In some implementations, the ligation reaction of step (2) uses a T4 RNA ligase 1. In some implementations, the elongase used in step (3) is a reverse transcriptase and/or Taq enzyme. In some implementations, the reverse transcriptase is an MMLV reverse transcriptase.

According to one aspect of the present invention, a method for processing a sample containing a DNA-RNA-DNA:cDNA heterozygous chain and a DNA:cDNA double strand is provided, the method comprises adding a DNA endonuclease to the sample, and a DNA:cDNA pairing region of the DNA:cDNA double strand contains a sequence of a recognition site of the DNA endonuclease. In some implementations, an RNA:cDNA pairing region of the DNA-RNA-DNA:cDNA heterozygous chain contains the sequence of the recognition site. In some implementations, the DNA-RNA-DNA:cDNA heterozygous chain is generated during construction of an RNA sequencing library. In some implementations, the DNA:cDNA double strand comprises a self-linking product formed by a 5' end linker and a 3' end linker used during RNA sequencing library construction. In some implementations, when the self-linking product is formed, the sequence of the recognition site of the DNA endonuclease is formed at the junction of the 5' end linker and the 3' end linker.

In some implementations, the RNA molecule in the sample is a small RNA molecule. In some implementations, the small RNA molecule has a length of 15-200 nt.

In some implementations, a content of the RNA molecules in the sample is ≤ 100 pg, ≤ 200 pg, ≤ 500 pg, ≤ 1 ng, ≤ 2 ng, ≤ 5 ng, ≤ 10 ng, or ≤ 20 ng. In some implementations, the content of the RNA molecule in the sample is ≥ 50 pg. In some implementations, the content of the RNA molecule in the sample is 50 pg-20 ng.

In some implementations, the method can efficiently remove the self-linking product formed by the 5' end linker and the 3' end linker in a system. In some implementations, the method can remove at least 50%, at least 60%, at least 70%, at least 80%, at least 90%, at least 95%, at least 98%, at least 99%, at least 99.5%, at least 99.8%, or at least 99.9% of the self-linking products in the system.

In some implementations, the 5' end of the 3' end linker carries at least one, at least two, at least three, or at least four consecutive nucleotides of the 3' end of the recognition site of the DNA endonuclease. In some implementations, the 5' end of the 3' end linker has an adenylation modification. In some implementations, the 3' end of the 3' end linker has a dideoxy modification. In some implementations, the 3' end linker is a DNA linker.

In some implementations, the 3' end of the 5' end linker carries at least one, at least two, at least three, or at least four consecutive nucleotides of the 5' end of the recognition site of the DNA endonuclease. In some implementations, the 5' end linker is a DNA linker.

In some implementations, neither the 5' end linker nor the 3' end linker comprises a complete sequence of the recognition site of the DNA endonuclease.

In some implementations, the DNA endonuclease can effectively cleave a DNA double strand containing the sequence of the recognition site thereof, but has low activity for an RNA:DNA heterozygous chain containing the sequence of the recognition site. In some implementations, a cleavage efficiency of the DNA endonuclease for an RNA:cDNA heterozygous chain containing the sequence of the recognition site thereof is at most 1/10, at most 1/100, at most 1/1000, or at most 1/10000 of a cleavage efficiency for the corresponding DNA:cDNA double strand, and the DNA:cDNA double strand contains a cDNA of the same sequence and a DNA complementary thereto. In some implementations, the DNA endonuclease is inactive, or has no detectable activity, for the RNA:cDNA heterozygous chain containing the sequence of the recognition site thereof.

In some implementations, the DNA endonuclease is a double-stranded DNA endonuclease. In some implementations, the DNA endonuclease is selected from the group of: AatII, BamHI, BsaBI, BsrFI, DraI, HphI, NdeI, PauI, PvuII, SwaI, Acc651, BanI, BsaHI, BsrGI, DraIII, Hpy188I, NgoMI, RsaI, TaqI, AccI, BanII, BsaI, BsrI, DrdI, Hpy188III, NheI, RsrII, TfiI, AciI, BbsI, BsaJi, BssHI, BssHII, EaeI, Hpy99I, NlaIII, SacI, TliI, AclI, BbvCI, BsaWI, BssKI, EagI, HpyCH4III, NlaIV, SacII, TseI, AcuI, BbvI, BsaXI, BssSI, EarI, HpyCH4IV, NotI, SalI, Tsp451, AfeI, BccI, BseRI, BstAPI, EciI, HpyCH4V, NruI, SapI, Tsp509I, AflII, BceAI, BseYI, BstBI, EcoNI, KasI, NsiI, Sau3AI, TspRI, AflIII, BcgI, BsgI, BstEII, EcoO109I, KpnI, NspI, Sau96I, Tth111I, AgeI, BciVI, BsiEI, BstF5I, EcoRI, MboI, PacI, Sbfl, Xbal, AhdI, Bell, BsiHKAI, BstNI, EcoRV, MboII, PaeR7I, Seal, XcmI, AleI, BfaI, BsiWI, BstUI, FatI, MfeI, PciI, ScrFI, XhoI, AluI, BfrBI, BsiI, BstXI, Paul, MluI, PflFI, SexAI, XmaI, AlwI, BfuAI, BsmAI, BstYI, Fnu4HI, MlyI, PflMI, SfaNI, XmnI, AlwNI, BfuCI, BsmBI, BstZ17I, FseI, MmeI, PhoI, SfcI, ZraI, ApaI, BglI, BsmFI, Bsu36I, FspI, MnlI, PleI, SfoI, ApaLI, BglII, BsmI, BtgI, HaeII, MscI, PmeI, SgrAI, Nb.BbvCI, ApeKI, BlpI, BsoBI, BtgZI, HaeIII, MseI, PmlI, SmaI, Nt.BbvCI, ApoI, Bme1580I, Bsp1286I, BtsI, HgaI, MsiI, PpuMI, SmlI, Nb.BsmI, AscI, BmgBI, BspCNI, Cac8I, HhaI, MspA1I, PshAI, SnaBI, Nt.BstNBI, AseI, BmrI, BspDI, ClaI, HincII, MspI, PsiI, SpeI, AsiSI, BmtI, BspEI, CspCI, HindIII, MwoI, PspGI, SphI, AvaI, BpmI, BspHI, CviAII, Hinfl, NaeI, PspOMI, SspI, Avail, Bpu10I, BspMI, DdeI, HinP1I, NarI, PspXI, StuI, AvrII, BpuEI, BsrBI, DpnI, HpaI, NciI, PstI, StyD4I, BaeI, BsaAI, BsrDI, DpnII, HpaII, Ncol, PvuI, and StyI. In some implementations, the double-stranded DNA endonuclease is not Avail, AvrII, BanI, HaeIII, Hinfl, TaqI, or other enzymes that can cleave a DNA/RNA heterozygous chain under specific conditions.

According to one aspect of the present invention, a method for determining sequence information of a small RNA molecule is provided, comprising: constructing a sequencing library based on a small RNA molecule sample by the method described herein; sequencing the sequencing library to obtain a sequencing result; and determining the sequence information of the small RNA molecule based on the sequencing result.

According to one aspect of the present invention, a system for determining sequence information of an RNA molecule is provided, comprising: a kit described herein; a sequencing device, used for sequencing the sequencing library constructed for a sample by the kit to obtain a sequencing result of the sample; and an analysis device, for analyzing the sequencing result to determine the sequence information of the RNA molecule.

In some implementations, the RNA molecule is a small RNA molecule.

According to one aspect of the present invention, use of the method described herein, or the kit described herein, or the system described herein in construction of an RNA sequencing library is provided. In some implementations, the RNA sequencing library is selected from the group of: a plasma small RNA sequencing library, a CLIP library, an RIP library, an MeRIP library, and a GRO library.

According to one aspect of the present invention, a method for constructing a sequencing library is provided, comprising the steps of:
(1) connecting a specific tag linker to the 3' terminus of an RNA molecule in a mixed sample of DNA and RNA molecules;
(2) obtaining a single-stranded DNA molecule using a reverse transcription primer based on the RNA molecule connected with the tag linker; and
(3) constructing a library for all DNA molecules in the sample.

According to one aspect of the present invention, a method for simultaneous sequencing of DNA and RNA molecules in a sample is provided, comprising the steps of:
(1) connecting a specific tag linker to the 3' terminus of an RNA molecule in a mixed sample of DNA and RNA molecules;
(2) obtaining a single-stranded DNA molecule using a reverse transcription primer based on the RNA molecule connected with the tag linker;
(3) constructing a library for all DNA molecules in the sample; and
(4) sequencing the library, and using a sequence of the specific tag linker to distinguish the RNA molecules in the mixed sample.

In some implementations, a ligase in the ligation reaction of step (1) is a truncated T4 RNA ligase 2 or a point mutant thereof.

In some implementations, a library construction method used in step (3) comprises: (a) denaturing the DNA molecule into a single strand; and (b) connecting specific linkers to the 3' terminus and/or 5' terminus of the single strand obtained in step (a). In some implementations, the library construction method used in step (3) further comprises (c) using an amplification primer for amplification to obtain an amplification product. In some implementations, the library construction method used in step (3) further comprises: (d) extending the product obtained in step (b) into a double-stranded DNA; and (e) connecting double-stranded linkers to one or both ends of the double-stranded DNA obtained in step (d). In some implementations, the library construction method used in step (3) further comprises (f) using an amplification primer for amplification to obtain an amplification product.

In some implementations, the library construction method used in step (3) comprises: (A) extending the single-stranded DNA molecule obtained in step (2) into a double-stranded DNA; and (B) connecting double-stranded linkers to one or both ends of the double-stranded DNA obtained in step (A). In some implementations, the library construction method used in step (3) further comprises (C) using an amplification primer for amplification to obtain an amplification product.

In some implementations, a sequencing method used in step (4) comprises using next-generation high-throughput sequencing or third-generation sequencing.

In some implementations, the 5' end of the specific tag linker has an adenylation modification. In some implementations, the 3' end of the specific tag linker has a dideoxy modification. In some implementations, the specific tag linker is a DNA linker.

In some implementations, the ligase used in the ligation reaction in step (1) can connect the specific linker to the 3' terminus of the RNA molecule but cannot connect the specific linker to the 3' terminus of the DNA molecule.

According to one aspect of the present invention, a method for determining sequence information of DNA and RNA molecules in a mixed sample of the DNA and RNA molecules is provided, comprising: based on the mixed sample of the DNA and RNA molecules, constructing a sequencing library by the method described herein and performing sequencing to obtain a sequencing result; and based on the sequencing result, determining the sequence information of the DNA and RNA molecules in the mixed sample of the DNA and RNA molecules.

According to one aspect of the present invention, a method for determining sequence information of a DNA target region being transcribed and a synthesized RNA molecule in a transcription complex is provided, comprising:
(i) obtaining a DNA and RNA sample of the region being transcribed;
(ii) constructing a sequencing library for the sample by the method described herein and performing sequencing to obtain a sequencing result;
(iii) based on the sequencing result, determining the sequence information of the DNA target region being transcribed and the synthesized RNA molecule.

In some implementations, step (i) comprises: randomly fragmenting chromatin in the sample, and performing co-immunoprecipitation on the chromatin using an antibody for an RNA polymerase, to obtain the DNA and RNA sample of the region being transcribed.

In some implementations, the randomly fragmented chromatin has an average length of 200 bp-500 bp.

In some implementations, step (i) comprises degrading proteins using proteinase K after the co-immunoprecipitation.

In some implementations, the sample contains a cell-free DNA and cell-free RNA.

According to one aspect of the present invention, a kit for constructing a sequencing library of DNA and RNA molecules in the same sample is provided, comprising: an RNA terminus connecting module, which comprises a specific tag linker and is used for connecting the tag linker to the 3' terminus of the RNA molecule.

In some implementations, the kit further comprises: a reverse transcription module, which comprises a reverse transcription primer and a reverse transcriptase and is used for generating a DNA molecule based on the RNA molecule connected with the tag linker.

In some implementations, the kit further comprises: a DNA library construction module, which is used for constructing the DNA molecule in the sample into the sequencing library.

In some implementations, the RNA terminus connecting module comprises a ligase. In some implementations, the ligase is a truncated T4 RNA ligase 2 or a point mutant thereof.

In some implementations, the 5' end of the specific tag linker has an adenylation modification. In some implementations, the 3' end of the specific tag linker has a dideoxy modification. In some implementations, the specific tag linker is a DNA linker.

According to one aspect of the present invention, a system for determining sequence information of DNA and RNA molecules is provided, comprising: the kit described herein; a sequencing device, used for sequencing the sequencing library constructed for a sample by the kit to obtain a sequencing result of the sample; and an analysis device, for analyzing the sequencing result of the sample to obtain the sequence information of the DNA and RNA molecules.

According to one aspect of the present invention, use of the method described herein, or the kit described herein, or the system described herein in simultaneous sequencing of DNA and RNA molecules in the same sample is provided.

Unless defined otherwise, all technical and scientific terms used herein have the same meanings as commonly understood by those skilled in the art to which the present invention belongs.

If nucleotide sequences between different DNA fragments or between a DNA fragment and an RNA fragment can be complementary to each other and can be renatured to form a new double helix structure, the process of combining two polynucleotide chains that are incompletely complementary to each other according to complementary base pairing is referred to as "molecular hybridization" or "molecular heterozygosis." The obtained polynucleotide double strand is referred to as a hybrid chain or heterozygous chain.

In some implementations, the heterozygous chain is a DNA-RNA-DNA:cDNA heterozygous chain including an RNA:cDNA pairing region. In some implementations, a DNA:cDNA double strand refers to a polynucleotide double strand formed by a DNA single strand and a cDNA single strand according to complementary base pairing. In some implementations, the DNA-RNA-DNA:cDNA heterozygous chain is generated during construction of an RNA sequencing library. In some implementations, the DNA in the DNA:cDNA double strand is a self-linking product formed by a 5' end linker and a 3' end linker used during RNA sequencing library construction.

"Nucleic acid endonucleases" refer to enzymes that can hydrolyze phosphodiester bonds in a nucleic acid molecular chain to generate oligonucleotides, and can be divided into DNA endonucleases decomposing the DNA and RNA endonucleases decomposing the RNA. The DNA endonucleases include "DNA restriction endonucleases," and the latter, which are also referred to as restriction endonucleases or restriction enzymes for short, refer to a class of active proteins that recognize specific nucleotide sequences and cleave a phosphodiester bond between two nucleotides at a specific site of each nucleic acid strand. The restriction endonucleases are widely distributed, and at least one type of restriction endonuclease is found in almost all genera and species of bacteria. Common DNA endonucleases include but are not limited to the DNA endonucleases listed in the following table:

**Table I. Common DNA endonucleases**

| |
|---|
| AatII, BamHI, BsaBI, BsrFI, DraI, HphI, NdeI, PauI, PvuII, SwaI, Acc65I, BanI, BsaHI, BsrGI, DraIII, Hpy188I, NgoMI, RsaI, TaqI, AccI, BanII, BsaI, BsrI, DrdI, Hpy188III, NheI, RsrII, TfiI, AciI, BbsI, BsaJi, BssHI, BssHII, EaeI, Hpy99I, NlaIII, SacI, TliI, AclI, BbvCI, BsaWI, BssKI, EagI, HpyCH4III, NlaIV, SacII, TseI, AcuI, BbvI, BsaXI, BssSI, EarI, HpyCH4IV, NotI, SalI, Tsp451, AfeI, BccI, BseRI, BstAPI, EciI, HpyCH4V, NruI, SapI, Tsp509I, AflII, BceAI, BseYI, BstBI, EcoNI, KasI, NsiI, Sau3AI, TspRI, AflIII, BcgI, BsgI, BstEII, EcoO109I, KpnI, NspI, Sau96I, Tth111I, AgeI, BciVI, BsiEI, BstF5I, EcoRI, MboI, PacI, SbfI, XbaI, AhdI, Bell, BsiHKAI, BstNI, EcoRV, MboII, PaeR7I, SeaI, XcmI, AleI, BfaI, BsiWI, BstUI, FatI, MfeI, PciI, ScrFI, XhoI, AluI, BfrBI, BsiI, BstXI, Paul, MluI, PflFI, SexAI, XmaI, AlwI, BfuAI, BsmAI, BstYI, Fnu4HI, MlyI, PflMI, SfaNI, XmnI, AlwNI, BfuCI, BsmBI, BstZ17I, FseI, MmeI, PhoI, SfcI, ZraI, ApaI, BglI, BsmFI, Bsu36I, FspI, MnII, PleI, SfoI, ApaLI, BglII, BsmI, BtgI, HaeII, MscI, PmeI, SgrAI, Nb.BbvCI, ApeKI, BlpI, BsoBI, BtgZI, HaeIII, MseI, PmlI, SmaI, Nt.BbvCI, ApoI, Bme1580I, Bsp1286I, BtsI, HgaI, MsiI, PpuMI, SmlI, Nb.BsmI, AscI, BmgBI, BspCNI, Cac8I, HhaI, MspA1I, PshAI, SnaBI, Nt.BstNBI, AseI, BmrI, BspDI, ClaI, HincII, MspI, PsiI, SpeI, AsiSI, BmtI, BspEI, CspCI, HindIII, MwoI, PspGI, SphI, AvaI, BpmI, BspHI, CviAII, HinfI, NaeI, PspOMI, SspI, AvaII, Bpu10I, BspMI, DdeI, HinP1I, NarI, PspXI, StuI, AvrII, BpuEI, BsrBI, DpnI, HpaI, NciI, PstI, StyD4I, BaeI, BsaAI, BsrDI, DpnII, HpaII, NcoI, PvuI, StyI |

Each of them can recognize a specific sequence and cleave the DNA sequence in this sequence in a specific manner. This specific sequence that the DNA endonuclease can recognize and cleave is also referred to as a "recognition site" or an "enzyme cleavage site." As long as there is a recognition site for a certain DNA endonuclease on the DNA sequence, the enzyme can be used to cleave the DNA sequence so as to perform various molecular biology operations.

"Gene sequencing" refers to analyzing the arrangement manner of base sequences in DNA or RNA fragments through certain technical analysis means, thereby providing support for biological and medical research discoveries.

The "sequencing library" refers to a collection of all DNA or RNA random fragments in a species or sample used for gene sequencing.

DNA (that is, deoxyribonucleic acid) refers to a type of nucleic acid that carries genetic information necessary for the synthesis of RNAs and proteins in biological cells, and is a biological macromolecule essential for growth and normal functioning of organisms. DNA is a macromolecular polymer composed of deoxyribonucleotides. The deoxyribonucleotides are composed of bases, deoxyriboses, and phosphoric acids, wherein there are four types of bases, being adenine (A), guanine (G), cytosine (C), and thymine (T), respectively. RNA (that is, ribonucleic acid) refers to a carrier of genetic information in biological cells, some viruses, and viroids. RNA is a macromolecular polymer composed of ribonucleotides. The ribonucleotides are composed of bases, riboses, and phosphoric acids; wherein there are four types of bases, being adenine (A), guanine (G), cytosine (C), and uracil (U), respectively.

The 5' end of the DNA or RNA refers to the terminus of a DNA or RNA single strand with a free 5'-hydroxyl group or a phosphate ester thereof. The 3' end of the DNA or RNA refers to the terminus of a DNA or RNA single strand with a free 3'-hydroxyl group or a phosphate ester thereof. In the present invention, the cDNA refers to a DNA strand complementary to an RNA after reverse transcription in vitro.

### Small RNAs

In the present invention, small RNAs refer to RNA molecules of 15-300 nt in length, preferably 15-200 nt, and more preferably 20-200 nt, including: (1) an microRNA (that is, miRNA), which refers to a type of endogenous small RNAs of about 20-24 nucleotides in length, and is generated from a hairpin-structured single-stranded RNA precursor of about 70-90 bases in size after processing by a Dicer enzyme; the microRNA has various important regulatory effects in cells; and each miRNA can have a plurality of target genes, while several miRNAs can also regulate the same gene; (2) a small interfering RNA (that is, siRNA, sometimes also referred to as a short interfering RNA or silencing RNA), which refers to a type of double-stranded RNAs of about 20-25 nucleotides in length, and is mainly involved in RNA interference phenomena and regulates gene expression in a specific manner; (3) a piwi-interacting RNA (that is, piRNA), which refers to a type of small RNA molecules of about 29-30 nucleotides in length; it mainly exists in germ cells and stem cells of mammals, and regulates a gene silencing pathway through binding to a Piwi subfamily protein to form a piRNA complex (piRC); (4) a small nuclear RNA (that is, snRNA), which refers to a main component of an RNA spliceosome in eukaryotic post-transcriptional processing, and is involved in the processing of an mRNA precursor; (5) a small nucleolar RNA (that is, snoRNA), which refers to a small-molecule non-coding RNA encoded by introns and distributed in nucleoli of eukaryotic cells, and has conserved structural elements; and antisense snoRNAs have been proven to guide methylation of rRNA riboses; and (6) a small ribosomal RNA (that is, srRNA), which refers to a small-sized ribosomal RNA that is mainly distributed in a coding region of an rDNA and matches a sense strand; and it has been found that the srRNA can specifically bind to an AGO protein, and its expression profile is related to diabetes.

In some implementations, the small RNAs include the microRNA (miRNA), the small interfering RNA (siRNA), the piwi-interacting RNA (piRNA), the small nuclear RNA (snRNA), the small nucleolar RNA (snoRNA), the small ribosomal RNA (srRNA), or any combination thereof.

A small RNA sample is obtained, and the small RNA sample can be used for constructing an RNA sequencing library. In some implementations, the small RNAs can be quantified by using a Qubit microRNA Assay Kit and a Qubit 4.0 Fluorometer.

In some implementations, the small RNA has a length of no more than 30 nt, no more than 40 nt, no more than 50 nt, no more than 70 nt, no more than 100 nt, no more than 150 nt, no more than 200 nt, no more than 250 nt, or not more than 300 nt. In some implementations, the small RNA has a length of at least 15 nt, at least 20 nt, or at least 30 nt. In some implementations, the small RNA has a length of 15-20 nt, 20-30 nt, 30-40 nt, 40-50 nt, 50-70 nt, 70-100 nt, 100-150 nt, 150-200 nt, 200-250 nt, 250-300 nt, 15-30 nt, 20-40 nt, 30-50 nt, 40-70 nt, 50-100 nt, 70-150 nt, 100-200 nt, 150-250 nt, 200-300 nt, 15-40 nt, 20-50 nt, 30-70 nt, 40-100 nt, 50-150 nt, 70-200 nt, 100-250 nt, 150-300 nt, 15-50 nt, 20-70 nt, 30-100 nt, 40-150 nt, 50-200 nt, 70-250 nt, 100-300 nt, 15-70 nt, 20-100 nt, 30-150 nt, 40-200 nt, 50-250 nt, 70-300 nt, 15-100 nt, 20-150 nt, 30-200 nt, 40-250 nt, 50-300 nt, 15-150 nt, 20-200 nt, 30-250 nt, 40-300 nt, 15-200 nt, 20-250 nt, 30-300 nt, 15-250 nt, 20-300 nt, or 15-300 nt.

In some implementations, a content of the small RNA sample is ≥ 1 pg, ≥ 2 pg, ≥ 5 pg, ≥ 10 pg, ≥ 20 pg, ≥ 50 pg, ≥ 100 pg, ≥ 200 pg, ≥ 500 pg, ≥ 1 ng, ≥ 2 ng, ≥ 5 ng, ≥ 10 ng, or ≥ 20 ng. In some implementations, the content of the small RNA sample is ≤ 1 pg, ≤ 2 pg, ≤ 5 pg, ≤ 10 pg, ≤ 20 pg, ≤ 50 pg, ≤ 100 pg, ≤ 200 pg, ≤ 500 pg, ≤ 1 ng, ≤ 2 ng, ≤ 5 ng, ≤ 10 ng, ≤ 20 ng, ≤ 50 ng, or ≤ 100 ng. In some implementations, the content of the small RNA sample is 1 pg-10 pg, 2 pg-20 pg, 5 pg-50 pg, 10 pg-100 pg, 20 pg-200 pg, 50 pg-500 pg, 100 pg-1 ng, 200 pg-2 ng, 500 pg-5 ng, 1 ng-10 ng, 2 ng-20 ng, 5 ng-50 ng, 10 ng-100 ng, 20 ng-200 ng, 1 pg-20 pg, 2 pg-50 pg, 5 pg-100 pg, 10 pg-200 pg, 20 pg-500 pg, 50 pg-1 ng, 100 pg-2 ng, 200 pg-5 ng, 500 pg-10 ng, 1 ng-20 ng, 2 ng-50 ng, 5 ng-100 ng, 10 ng-200 ng, 1 pg-50 pg, 2 pg-100 pg, 5 pg-200 pg, 10 pg-500 pg, 20 pg-1 ng, 50 pg-2 ng, 100 pg-5 ng, 200 pg-10 ng, 500 pg-20 ng, 1 ng-50 ng, 2 ng-100 ng, 5 ng-200 ng, 1 pg-100 pg, 2 pg-200 pg, 5 pg-500 pg, 10 pg-1 ng, 20 pg-2 ng, 50 pg-5 ng, 100 pg-10 ng, 200 pg-20 ng, 500 pg-50 ng, 1 ng-100 ng, 2 ng-200 ng, 1 pg-200 pg, 2 pg-500 pg, 5 pg-1 ng, 10 pg-2 ng, 20 pg-5 ng, 50 pg-10 ng, 100 pg-20 ng, 200 pg-50 ng, 500 pg-100 ng, 1 ng-200 ng, 1 pg-500 pg, 2 pg-1 ng, 5 pg-2 ng, 10 pg-5 ng, 20 pg-10 ng, 50 pg-20 ng, 100 pg-50 ng, 200 pg-100 ng, 500 pg-200 ng, 1 pg-1 ng, 2 pg-2 ng, 5 pg-5 ng, 10 pg-10 ng, 20 pg-20 ng, 50 pg-50 ng, 100 pg-100 ng, 200 pg-200 ng, 1 pg-2 ng, 2 pg-5 ng, 5 pg-10 ng, 10 pg-20 ng, 20 pg-50 ng, 50 pg-100 ng, 100 pg-200 ng, 1 pg-5 ng, 2 pg-10 ng, 5 pg-20 ng, 10 pg-50 ng, 20 pg-100 ng, 50 pg-200 ng, 1 pg-10 ng, 2 pg-20 ng, 5 pg-50 ng, 10 pg-100 ng, 20 pg-200 ng, 1 pg-20 ng, 2 pg-50 ng, 5 pg-100 ng, 10 pg-200 ng, 1 pg-50 ng, 2 pg-100 ng, 5 pg-200 ng, 1 pg-100 ng, 2 pg-200 ng, or 1 pg-200 ng. In some implementations, the content of the small RNA sample is 50 pg-20 ng.

### 3 ' end linker and 5 ' end linker for an RNA sample

Connecting the RNA sample with the 3' end linker refers to connecting the 3' end linker to the 3' end of the RNA sample molecule. The 3' end A linker of the present invention refers to any short nucleic acid sequence capable of being connected with the 3' terminus of the RNA molecule. In some implementations, the 3' end linker is a DNA linker.

In some implementations, the linker is a short nucleic acid sequence connected with the 3' terminus of the RNA molecule through a truncated T4 RNA ligase 2 or a point mutant thereof. The enzyme can only connect an adenylated single-stranded DNA or RNA linker to the 3' terminus of the RNA molecule, thereby avoiding self-linking between the RNA molecules. In some implementations, the linker has an adenylation modification at the 5' end and a dideoxy modification at the 3' end, thereby preventing self-linking of the linker during ligation reaction. In some implementations, the 5' end of the 3' end linker carries a partial sequence of a recognition site of a corresponding DNA endonuclease, for example, one, two, three, four, or more than four consecutive nucleotides of the 3' end of the recognition site of the corresponding DNA endonuclease. In some implementations, the 5' end of the 3' end linker carries at least one nucleotide of the 3' end of the recognition site of the corresponding DNA endonuclease. In some implementations, the 5' end of the 3' end linker carries at least two consecutive nucleotides of the 3' end of the recognition site of the corresponding DNA endonuclease. In some implementations, the 5' end of the 3' end linker carries at least three consecutive nucleotides of the 3' end of the recognition site of the corresponding DNA endonuclease. In some implementations, the 5' end of the 3' end linker carries at least four consecutive nucleotides of the 3' end of the recognition site of the corresponding DNA endonuclease.

In some implementations, the 5' end of the 3' end linker has an adenylation modification, and the 3' end has a dideoxy modification. In some implementations, the modifications prevent self-linking of the linker during the ligation reactions.

In some implementations, the 3' end linker contains at least 10, at least 15, at least 20, at least 25, or at least 30 nucleotides. In some implementations, the 3' end linker contains no more than 15, no more than 20, no more than 25, no more than 30, no more than 35, no more than 40, no more than 45, or no more than 50 nucleotides. In some implementations, the 3' end linker contains 10-15, 10-20, 10-25, 10-30, 10-35, 10-40, 10-45, 10-50, 15-20, 15-25, 15-30, 15-35, 15-40, 15-45, 15-50, 20-25, 20-30, 20-35, 20-40, 20-45, 20-50, 25-30, 25-35, 25-40, 25-45, 25-50, 30-35, 30-40, 30-45, or 30-50 nucleotides.

In some implementations, the sequence of the 3' end linker may be, for example, 5'-rAPP-**CGC**AAGTCGGAGGCCAAG-3'ddC (SEQ ID NO: 1) (wherein the bold CGC are three consecutive nucleotides of the 3' end of a BssHII endonuclease).

In some implementations, the ligation can be performed in a reaction system including the following: the RNA molecule sample, the 3' end linker, and the truncated T4 RNA ligase 2. In some implementations, the reaction system further includes a ligase reaction buffer, PEG8000, and an RNA enzyme inhibitor. In some implementations, the RNA molecule sample is subjected to thermal denaturation treatment (for example, reacting at 70°C for 2 minutes). In some implementations, the ligation can be performed under the following reaction conditions: at 16°C overnight, reacting at 25°C for 2 hours, or reacting at 37°C for 30 minutes.

Connecting the RNA molecule connected with the 3' end linker with the 5' end linker refers to connecting the 5' end linker to the 5' end of the RNA sample molecule connected with the 3' end linker. In some implementations, the 5' end linker refers to a short nucleic acid sequence capable of being connected with the 5' terminus of the RNA sample molecule. In some implementations, the 5' end linker is a DNA linker.

In some implementations, the 5' end linker is a short nucleic acid sequence connected with the 5' terminus of the RNA molecule through a T4 RNA ligase 1. In some implementations, the 3' end of the 5' end linker carries a partial sequence of a recognition site of a corresponding DNA endonuclease, for example, one, two, three, four, or more than four consecutive nucleotides of the 5' end of the recognition site of the corresponding DNA endonuclease. In some implementations, the 3' end of the 5' end linker carries at least one nucleotide of the 5' end of the recognition site of the corresponding DNA endonuclease. In some implementations, the 3' end of the 5' end linker carries at least two nucleotides of the 5' end of the recognition site of the corresponding DNA endonuclease. In some implementations, the 3' end of the 5' end linker carries at least three nucleotides of the 5' end of the recognition site of the corresponding DNA endonuclease. In some implementations, the 3' end of the 5' end linker carries at least four nucleotides of the 5' end of the recognition site of the corresponding DNA endonuclease.

In some implementations, the 5' end linker contains at least 10, at least 15, at least 20, at least 25, or at least 30 nucleotides. In some implementations, the 5' end linker contains no more than 15, no more than 20, no more than 25, no more than 30, no more than 35, no more than 40, no more than 45, or no more than 50 nucleotides. In some implementations, the 5' end linker contains 10-15, 10-20, 10-25, 10-30, 10-35, 10-40, 10-45, 10-50, 15-20, 15-25, 15-30, 15-35, 15-40, 15-45, 15-50, 20-25, 20-30, 20-35, 20-40, 20-45, 20-50, 25-30, 25-35, 25-40, 25-45, 25-50, 30-35, 30-40, 30-45, or 30-50 nucleotides.

In some implementations, the sequence of the 5' end linker may be, for example, 5'-GCTACGATCCGACTTNNNNGCG-3' (SEQ ID NO: 2) (wherein the bold GCG are three consecutive nucleotides of the 5' end of the BssHII endonuclease).

In some implementations, the ligation can be performed in a reaction system including the following: the RNA sample molecule connected with the 3' end linker, the 5' end linker, and the T4 RNA ligase 1. In some implementations, the reaction system further includes a ligase reaction buffer and PEG8000. In some implementations, the ligation can be performed under the following reaction conditions: at 16°C overnight or reacting at 25°C for 2 hours.

In the application of DNA and RNA library co-construction, connecting the RNA molecule with a specific tag linker refers to connecting the specific tag linker to the 3' terminus of the RNA molecule. The specific tag linker of the present invention refers to any short nucleic acid sequence capable of being connected with the 3' terminus of the RNA molecule. In some implementations, the specific tag linker is a DNA linker.

In some implementations, the linker is a short nucleic acid sequence connected with the 3' terminus of the RNA molecule through a truncated T4 RNA ligase 2 or a point mutant thereof. The enzyme can only connect an adenylated single-stranded DNA or RNA linker to the 3' terminus of the RNA molecule, but not to the 3' terminus of a DNA molecule, thereby achieving a specific connection with the RNA molecule. In some implementations, the linker has an adenylation modification at the 5' end and a dideoxy modification at the 3' end, thereby preventing self-linking of the linker and self-linking between the DNA and RNA molecules during the ligation reactions. In some implementations, a sequence of n arbitrary bases can also be introduced at the 5' end of the linker to correct PCR amplification deviations that may be caused when constructing a library with a low starting amount, where n may be a positive integer from 1 to 15. In some implementations, n is 1, 2, 3, 4, 5, 6, 7, 8, 9, 10, 11, 12, 13, 14, or 15. In some implementations, n is at least 1, at least 2, at least 3, at least 4, at least 5, at least 6, at least 7, at least 8, at least 9, at least 10, at least 11, at least 12, at least 13, or at least 14. In some implementations, n is no more than 2, no more than 3, no more than 4, no more than 5, no more than 6, no more than 7, no more than 8, no more than 9, no more than 10, no more than 11, no more than 12, not more than 13, not more than 14, or not more than 15.

In some implementations, the 5' end of the specific tag linker has an adenylation modification, and the 3' end has a dideoxy modification. In some implementations, the modifications prevent self-linking of the linker and self-linking between the DNA and RNA molecules during the ligation reactions.

In some implementations, the specific tag linker contains at least 10, at least 15, at least 20, at least 25, or at least 30 nucleotides. In some implementations, the specific tag linker contains no more than 15, no more than 20, no more than 25, no more than 30, no more than 35, no more than 40, no more than 45, or no more than 50 nucleotides. In some implementations, the specific tag linker contains 10-15, 10-20, 10-25, 10-30, 10-35, 10-40, 10-45, 10-50, 15-20, 15-25, 15-30, 15-35, 15-40, 15-45, 15-50, 20-25, 20-30, 20-35, 20-40, 20-45, 20-50, 25-30, 25-35, 25-40, 25-45, 25-50, 30-35, 30-40, 30-45, or 30-50 nucleotides.

The sequence of the specific tag linker may be, for example, 5'-rAPP-(N)nTGATGCGTAGCCTTAA-3'ddC (SEQ ID NO: 3).

In some implementations, the ligation can be performed in a reaction system including the following: a sample of the DNA and RNA molecules, the specific tag linker, and the truncated T4 RNA ligase 2. In some implementations, the reaction system further includes a ligase reaction buffer, PEG8000, and an RNA enzyme inhibitor. In some implementations, the sample of the DNA and RNA molecules is subjected to thermal denaturation treatment. In some implementations, the ligation can be performed under the following reaction conditions: at 16°C overnight, reacting at 25°C for 2 hours, or reacting at 37°C for 30 minutes.

### Ligase at the 3' terminus of an RNA molecule

In some implementations, the method and kit of the present invention include a ligase that connects the 3' terminus of the RNA molecule to a corresponding linker (for example, a 3' end linker or a specific tag linker). In some implementations, the ligase can only connect an adenylated single-stranded DNA or RNA linker to the 3' terminus of the RNA molecule, but not to the 3' terminus of a DNA molecule, thereby achieving a specific connection with the RNA molecule.

In some implementations, the ligase is a truncated T4 RNA ligase 2 or a point mutant thereof. The selection of a suitable T4 RNA ligase 2 or a point mutant thereof is apparent to those skilled in the art. In some implementations, the truncated T4 RNA ligase 2 includes only 249 amino acids at the N-terminal of the T4 RNA ligase 2. In some implementations, the point mutant includes R55K and/or K227Q mutations. In some implementations, the point mutant pack is a double mutant of R55K and K227Q. Many biotechnology companies provide various truncated T4 RNA ligases 2 or point mutants thereof, such as New England Biolabs^{®}, Catalog Numbers M0242 and M0373.

### Library construction

Reverse transcription refers to a DNA synthesis process using an RNA as a template, that is, DNA synthesis under the guidance of the RNA. This process is referred to as reverse transcription because both the direction of nucleic acid synthesis and the flow direction of genetic information (RNA to DNA) in this process are opposite to that of the transcription process (DNA to RNA). In some implementations, the reverse transcription is to, under the action of a reverse transcriptase and using a dNTP as a substrate and the RNA as the template, extend the 3'-terminus of a reverse transcription primer along the 5'→3' direction to synthesize a cDNA single strand complementary to the RNA template, which forms an RNA-cDNA hybrid with the RNA template. In some implementations, the RNA strand can be further hydrolyzed under the action of the reverse transcriptase, and then a second DNA strand is synthesized using the cDNA as a template. At this point, the DNA synthesis process guided by the RNA is completed. The reverse transcription primer may adopt a random primer or a gene-specific primer. In some implementations, the reverse transcription primer is a sequence designed according to the known sequence of the specific tag linker of the present invention, which may be, for example, **5'-GTTAAGGCTACGCATCA-3'** (SEQ ID NO: 4) (wherein the sequence indicated in bold is a sequence that is reversely complementary to the sequence of the specific tag linker). In some implementations, the reverse transcription can be performed in a reaction system including the following: the RNA molecule connected with the specific tag linker, the reverse transcription primer, and the reverse transcriptase. In some implementations, the reaction system further includes a reverse transcriptase buffer and an RNA enzyme inhibitor. In some implementations, a temperature of the reverse transcription can be determined based on the optimal reaction temperature of the reverse transcriptase. In some implementations, the reverse transcription can be performed under the following reaction conditions: reacting at 42°C for 30 minutes. In some implementations, a product of the reverse transcription can be purified, for example, by using a magnetic beads method. In some implementations, dephosphorylation treatment can be performed on the purified product of the reverse transcription. In some implementations, the dephosphorylation treatment can be performed in a reaction system including an alkaline phosphatase. In some implementations, the reaction system may further include a reaction buffer. In some implementations, the dephosphorylation treatment can be performed under the following conditions: at 37°C for 30 minutes and at 95°C for 5 minutes to obtain a DNA single strand.

In some implementations, connecting a single-stranded linker with the DNA single strand refers to connecting the single-stranded linker to the 3' terminus and/or 5' terminus of the DNA single strand obtained in the previous step. The single-stranded linker of the present invention refers to any short nucleic acid sequence capable of being connected with the 3' terminus and/or 5' terminus of the DNA single strand. In some implementations, the single-stranded linker is a DNA linker.

In some implementations, the linker has a phosphorylation modification at the 5' end and a dideoxy modification at the 3' end, thereby preventing self-linking of the linker and self-linking between DNA molecules during the ligation reactions. In some implementations, a sequence of n arbitrary bases can also be introduced at the 5' end of the linker to correct PCR amplification deviations that may be caused when constructing a library with a low starting amount, where n may be a positive integer from 1 to 15. In some implementations, n is 1, 2, 3, 4, 5, 6, 7, 8, 9, 10, 11, 12, 13, 14, or 15. In some implementations, n is at least 1, at least 2, at least 3, at least 4, at least 5, at least 6, at least 7, at least 8, at least 9, at least 10, at least 11, at least 12, at least 13, or at least 14. In some implementations, n is no more than 2, no more than 3, no more than 4, no more than 5, no more than 6, no more than 7, no more than 8, no more than 9, no more than 10, no more than 11, no more than 12, not more than 13, not more than 14, or not more than 15.

The sequence of the single-stranded linker may be, for example, 5'-Pho-(N)nAAGTCGGATCGTAGCCATG-3'ddC (SEQ ID NO: 5). In some implementations, the ligation can be performed in a reaction system including the following: the DNA single strand obtained in the previous step, the single-stranded linker, and a T4 RNA ligase. In some implementations, the reaction system further includes a ligase reaction buffer and PEG8000. In some implementations, the ligation can be performed under the following reaction conditions: at 16°C overnight or reacting at 30°C for 2 hours.

In some implementations, an extension reaction refers to a process of using a ligation product as a template and using an extension primer designed according to the known sequence of the 3' end linker to perform extension and obtain an extension product. The extension primer is a sequence designed according to the known sequence of the 3' end linker of the present invention, which may be, for example, 5'-TTGTCTTCCTAAGACCGCTTGG**CCTCCGACTTGCG**-3' (SEQ ID NO: 6) (wherein the sequence indicated in bold is a sequence that is reversely complementary to the sequence of the 3' end linker).

In some implementations (for example, when constructing a sequencing library for simultaneous sequencing of the DNA and RNA), extension is a process of using the DNA single strand obtained by reverse transcription or the ligation product obtained in the previous step as a template and using an extension primer to perform extension and obtain a double-stranded DNA product. In some implementations, the extension primer is a sequence designed according to the known sequence of the single-stranded linker of the present invention, which may be, for example, 5'-CAACTCCTTGGCTCACAGAACGA**CATGGCTACGATCCGACTT**-3' (SEQ ID NO: 7) (wherein the sequence indicated in bold is a sequence that is reversely complementary to the sequence of the single-stranded linker). In some implementations, the extension can be performed in a reaction system including the following: the DNA single strand obtained by the reverse transcription or the ligation product obtained in the previous step, the extension primer, and a DNA polymerase. In some implementations, the reaction system further includes a DNA polymerase buffer. In some implementations, the extension can be performed under the following reaction conditions: at 95°C for 2 minutes, at 45°C for 30 seconds, and at 72°C for 5 minutes.

In some implementations, the extension can be performed in a reaction system including the following: the ligation product obtained in the previous step, the extension primer, the reverse transcriptase, and/or a Bst polymerase.

In some implementations, the extension can be performed in a reaction system including the following: the ligation product obtained in the previous step, the extension primer, the reverse transcriptase, and the DNA polymerase.

In some implementations, the reaction system further includes an extension reaction buffer. In some implementations, the ligation can be performed under the following reaction conditions: reacting at 42°C for 30 minutes.

In some implementations (for example, when constructing the sequencing library for simultaneous sequencing of the DNA and RNA), double-end linker ligation refers to a process of connecting a double-stranded linker with the double-stranded DNA product obtained in the previous step. The double-stranded linker connected with a double-stranded DNA molecule refers to in the present invention any short double-stranded nucleic acid sequence capable of being connected with the obtained double-stranded DNA molecule. In some implementations, the double-stranded linker is a DNA linker. In some implementations, the linker is composed of an F sequence and an R sequence, where the R sequence is reversely complementary to the F sequence. In some implementations, there is an extra base T at the end of the R sequence. In some implementations, the F sequence and the R sequence of the double-stranded linker may be, for example, 5'-Pho-**GAAGTCGGA**GGCCAAGCGGTCTTAGGAAGACAA-3' (SEQ ID NO:8) and 5'-CAACTCCTTGGCTCACAGAACGACATGGCTACGA**TCCGACTTCT**-3' (SEQ ID NO: 9), respectively, (the bold indicates reverse complementary regions of the F sequence and the R sequence). In some implementations, the double-end linker ligation can be performed in a reaction system including the following: the double-stranded DNA product obtained in the previous step, the double-stranded linker, and the T4 RNA ligase. In some implementations, the reaction system further includes a T4 DNA ligase buffer. In some implementations, the ligation can be performed under the following reaction conditions: reacting at 20°C for 30 minutes.

Enzyme cleavage treatment in the present invention refers to a process of cleaving the extension product by using an enzyme. When using a DNA endonuclease, reaction conditions for enzyme cleavage change depending on different DNA endonucleases used, and are usually 20-60°C, preferably 28-50°C, and more preferably 37-42°C.

In some implementations, the DNA endonuclease used is BssHII, and the enzyme cleavage condition adopted is incubating a sequence to be cleaved with the enzyme at 37°C for 30 minutes.

In some implementations, the DNA endonuclease used is Paul, and the enzyme cleavage condition adopted is incubating a sequence to be cleaved with the enzyme at 37°C for 30 minutes.

Second-strand synthesis refers to synthesizing a second-strand synthesis product using a second-strand synthesis primer based on a template of an enzyme cleavage product of the present invention. The second-strand synthesis primer is a sequence designed according to the known sequence of the 5' end linker sequence of the present invention, which may be, for example, 5'-CAACTCCTTGGCTCACAGAACGACATG**GCTACGATCCGACTT**-3' (SEQ ID NO: 10) (wherein the sequence indicated in bold is a 5' sequence of the sequence of the 5' end linker). In some implementations, the second-strand synthesis can be performed in a reaction system including the following: the enzyme cleavage product, the second-strand synthesis primer, and the DNA polymerase. In some implementations, the reaction system further includes a DNA polymerase buffer. In some implementations, the ligation can be performed under the following reaction conditions: at 98°C for 2 minutes, at 60°C for 30 seconds, and at 72°C for 5 minutes.

In some implementations, an enzyme capable of specifically hydrolyzing an RNA in a DNA-RNA heterozygous chain is further used in the enzyme cleavage reaction. In some implementations, the enzyme cannot digest a single- or double-stranded DNA. In some implementations, the enzyme cannot digest a single- or double-stranded DNA. In some implementations, the enzyme capable of specifically hydrolyzing the RNA in the DNA-RNA heterozygous chain is RNase H. The selection of a suitable enzyme capable of specifically hydrolyzing the RNA in the DNA-RNA heterozygous chain is apparent to those skilled in the art.

In some implementations, amplification or PCR amplification refers to: treating a target DNA double strand containing a required amplification analysis sequence at a high temperature for a period of time to form two oligonucleotide single strands (melting), and adding a pair of oligonucleotide fragments, which are artificially synthesized according to a known DNA sequence and complementary to sequences adjacent to both ends of the amplified DNA, as amplification primers, that is, forward and reverse primers. The primers are in contact with two DNA single strands at a low temperature for a period of time for respective complementary binding (annealing), and at a high temperature and under the action of the DNA polymerase, the primers are extended using a target DNA single strand as a template and four types of single nucleotides (dNTPs) as raw materials for a period of time in a direction from 5' to 3', so as to synthesize a new DNA double strand (extension). Then, another cycle of melting-annealing-extension begins, thereby specifically amplifying an extremely small amount of target DNA by a million times or more (that is, amplification product). In some implementations, the amplification of the present invention is synthesizing PCR reaction products using PCR primers based on the product obtained by the second-strand synthesis of the present invention. The PCR primers are sequences designed according to known sequences of the extension primer and the second-strand synthesis primer of the present invention, and in some implementations, there may be a certain number of random bases in the forward and reverse primers used for distinguishing different samples in high-throughput sequencing. The forward and reverse primers may be, respectively: for example, a forward primer of 5' -GCATGGCGACCTTATCAGNNNNNNNNNN**TTGTCTTCCTAAGACCGCTTGG-**3' (SEQ ID NO: 11) (wherein the sequence indicated in bold is a 5' sequence of the extension primer) and a reverse primer of 5'-Pho-CTCTCAGTACGTCAGCAGTTNNNNNNNNNN**CAACTCCTTGGCTCACAGAAC**-3' (SEQ ID NO: 12) (wherein the sequence indicated in bold is a 5' sequence of the second-strand synthesis primer), wherein 10 random bases are used for distinguishing different samples in the high-throughput sequencing. In some implementations, the PCR amplification can be performed in a reaction system including the following: the product obtained by the second-strand synthesis, the forward primer, the reverse primer, and the DNA polymerase. In some implementations, the DNA polymerase is a high-fidelity DNA polymerase. In some implementations, the reaction system further includes a DNA polymerase buffer and dNTPs. In some implementations, the ligation can be performed under the following reaction conditions: at 98°C for 2 minutes; then 15-30, preferably 15-20, and more preferably 15-18 cycles of at 98°C for 15 seconds, at 60°C for 30 seconds, and at 72°C for 30 seconds; and then reacting at 72°C for 5 minutes. In some implementations, PCR products are purified using a magnetic beads method.

In some implementations (for example, when constructing a sequencing library for simultaneous sequencing of the DNA and RNA), amplification or PCR amplification refers to: treating a target DNA double strand containing a required amplification analysis sequence at a high temperature for a period of time to form two oligonucleotide single strands (melting), and adding a pair of oligonucleotide fragments, which are artificially synthesized according to a known DNA sequence and complementary to sequences adjacent to both ends of the amplified DNA, as amplification primers, that is, forward and reverse primers. The primers are in contact with two DNA single strands at a low temperature for a period of time for respective complementary binding (annealing), and at a high temperature and under the action of the DNA polymerase, the primers are extended using a target DNA single strand as a template and four types of single nucleotides (dNTPs) as raw materials for a period of time in a direction from 5' to 3', so as to synthesize a new DNA double strand (extension). Then, another cycle of melting-annealing-extension begins, thereby specifically amplifying an extremely small amount of target DNA by a million times or more (that is, amplification product). In some implementations, the amplification of the present invention is synthesizing PCR reaction products using PCR primers based on the product obtained by the double-end ligation reaction of the present invention. The PCR primers are sequences designed according to the known sequences of the 5' end of the R sequence and the 3' end of the F sequence in the double-stranded linker sequence of the present invention. In some implementations, there may be a certain number of random bases in the forward and reverse primers used for distinguishing different samples in high-throughput sequencing. The forward and reverse primers may be, respectively, for example: the forward primer of 5'-GCATGGCGACCTTATCAG**TTGTCTTCCTAAGACCGCTTGG-3'** (SEQ ID NO: 11) (wherein the sequence indicated in bold is a sequence reversely complementary to the F sequence) and the reverse primer of 5'-Pho-CTCTCAGTACGTCAGCAGTTNNNNNNNNNN**CAACTCCTTGGCTCACAGAAC**-3' (SEQ ID NO: 12) (wherein the sequence indicated in bold is a 5' sequence of the R sequence), wherein there are 10 random bases used for distinguishing different samples in the high-throughput sequencing. In some implementations, the PCR amplification can be performed in a reaction system including the following: the product obtained by the double-end ligation reaction, the forward primer, the reverse primer, and the DNA polymerase. In some implementations, the DNA polymerase is a high-fidelity DNA polymerase. In some implementations, the reaction system further includes a DNA polymerase buffer. In some implementations, the PCR amplification can be performed under the following reaction conditions: at 98°C for 2 minutes; then 8-30, preferably 8-20, and more preferably 8-15 cycles of at 98°C for 15 seconds, at 60°C for 30 seconds, and at 72°C for 30 seconds; and then reacting at 72°C for 5 minutes. In some implementations, PCR products are purified using a magnetic beads method.

In some embodiments, the sequencing library of the present invention is applicable to next-generation high-throughput sequencing and/or third-generation sequencing.

In some embodiments, the sequencing is the next-generation high-throughput sequencing. The next-generation high-throughput sequencing refers to a technology that adopts microbeads or high-density chips for synthesis and sequencing at the same time, and its advantage is that the sequencing throughput is high and several gigabytes of data can be obtained at a time, but amplification is usually required to amplify sequencing signals.

In some embodiments, the sequencing is the third-generation sequencing. The third-generation sequencing refers to a single-molecule sequencing technology that usually adopts nanomaterials to directly sequence a single-stranded DNA/RNA in direct manners of synthesis, degradation, passing through nanopores, etc., and its advantage is that amplification is not required, so that the cost is lower.

To solve the foregoing technical problems existing in the prior art, an objective of the present invention is to provide a method for removing linker self-linking products during sequencing library construction. According to this method, the linker self-linking products can be specifically cleaved by utilizing the substrate selectivity of the DNA endonuclease, thereby achieving simple and efficient library construction of small RNAs or RNA fragments of micro samples.

To achieve the foregoing obj ective, the present invention adopts the following technical solutions:
According to one aspect of the present invention, a method for removing linker self-linking products during sequencing library construction is provided, and the method comprises: a step of mixing an DNA endonuclease with the DNA double strand and DNA single strand: wherein an enzyme cleavage site in the DNA double strand is located in a DNA:DNA pairing region; the DNA endonuclease can recognize the enzyme cleavage site in the DNA double strand and perform cleavage.

In some implementations, the DNA endonuclease can effectively cleave the DNA double strand containing the sequence of the recognition site thereof, but has low activity for a DNA single strand containing the sequence of the recognition site. In some implementations, the DNA double strand is generated during construction of the sequencing library. In some implementations, the DNA double strand is a self-linking product formed by a 5' end linker and a 3' end linker used during RNA sequencing library construction. In some implementations, both the 5' end linker and the 3' end linker contain a partial sequence of the recognition site, and when the linker self-linking product is formed, a complete sequence of the recognition site is formed at the junction of the 5' end linker and the 3' end linker. In some implementations, the sequencing library is an RNA sequencing library. In some implementations, the 3' end linker and the 5' end linker respectively carry more than 30%, preferably more than 40%, and more preferably 50% of the sequence of the recognition site of the nucleic acid endonuclease, and when the self-linking product is formed, the complete sequence of the recognition site is formed at the junction of the 5' end linker and the 3' end linker. In some implementations, the DNA endonuclease may be selected from the DNA endonucleases listed in Table I.

According to another aspect of the present invention, a method for removing 5' and 3' linker self-linking products generated during RNA sequencing library construction, or a method for constructing the RNA sequencing library is provided, comprising the steps of:
(1) connecting a 3' end DNA linker to the 3' terminus of an RNA molecule;
(2) connecting a 5' end DNA linker to the 5' terminus of the RNA molecule connected with the 3' end linker;
(3) obtaining an extension product using an extension primer and an elongase based on the RNA molecule connected with the 3' end linker and the 5' end linker;
(4) performing enzyme cleavage treatment on the extension product using RNase H and a DNA endonuclease, to remove the linker self-linking products in a system; and
(5) performing PCR amplification using primers related to a sequence of the linker, the obtained amplification product being the sequencing library.

In some implementations, the 3' end linker and the 5' end linker each contain a partial sequence of a recognition site of the nucleic acid endonuclease, for example, more than 30%, preferably more than 40%, and more preferably 50% of the sequence of the recognition site, and when the linker self-linking product is formed, a complete sequence of the recognition site is formed at the junction of the 5' end linker and the 3' end linker. In some implementations, a content of the RNA molecule is ≥ 50 pg. In some implementations, the content of the RNA molecule is 50 pg-20 ng. In some implementations, the RNA molecule is a small RNA molecule. In some implementations, the small RNA molecule has a length of 15-200 nt. In some implementations, a cleavage efficiency of the DNA endonuclease for a DNA single strand containing the sequence of the recognition site thereof is at most 1/10, at most 1/100, at most 1/1000, or at most 1/10000 of a cleavage efficiency for a corresponding DNA:DNA double strand, the DNA:DNA double strand contains the DNA single strand of the same sequence and a DNA single strand complementary thereto; and preferably, the DNA endonuclease is inactive, or has no detectable activity, for the DNA single strand containing the sequence of the recognition site thereof. In some implementations, the ligation reaction of step (1) uses a truncated T4 RNA ligase 2 as a ligase. In some implementations, the elongase used in step (3) is a reverse transcriptase or Bst polymerase. In some implementations, the DNA endonuclease used in step (4) is a double-stranded DNA endonuclease. In some implementations, the DNA endonuclease may be selected from the DNA endonucleases listed in Table I.

By using the method for removing the 5' and 3' linker self-linking by-products generated in the construction process of the sequencing library according to an implementation of the present invention, a sequencing library of small RNA molecules in a micro sample can be effectively constructed, and especially small RNA sequencing libraries of a plasma sample, a single cell sample, and other micro samples can be effectively constructed. Particularly, the library construction process provided in the present invention can optionally use a one-tube reaction process, further reducing an input amount in library construction, and reducing a deviation in the sequencing result caused by a purification process.

According to another aspect of the present invention, a kit for constructing a small RNA sequencing library is provided. According to an implementation of the present invention, the kit comprises: an RNA 3' terminus connecting module, wherein the connecting module comprises a 3' end linker and is used for connecting the 3' end linker to the 3' terminus of an RNA; an RNA 5' terminus connecting module, wherein the connecting module comprises a 5' end linker and is used for connecting the 5' end linker to the 5' terminus of the RNA connected with the 3' end linker; an extension module, wherein the extension module comprises an elongase and is used for extending the RNA molecule connected with the 3' end linker and the 5' end linker; an enzyme cleavage module, wherein the enzyme cleavage module comprises RNase H and a DNA endonuclease and is used for removing linker self-linking products in products of the extension module; and an amplification module, wherein the amplification module comprises an enzyme required for DNA amplification and is used for amplifying enzyme cleavage products, to obtain a final library. In some implementations, the extension module and the enzyme cleavage module can be integrated into one module, that is, an extension + enzyme cleavage module.

In some implementations, the RNA 3' terminus connecting module comprises a ligase. In some implementations, the ligase is a truncated T4 RNA ligase 2 or a point mutant thereof. In some implementations, the RNA 5' terminus connecting module comprises a ligase, and preferably, the ligase is a T4 RNA ligase 1. In some implementations, the elongase is an MMLV reverse transcriptase and/or Bst polymerase. In some implementations, the DNA endonuclease is a double-stranded DNA endonuclease. In some implementations, the DNA endonuclease may be selected from the DNA endonucleases listed in Table I. In some implementations, the DNA endonuclease is BssHII.

According to another aspect of the present invention, a method for removing linker self-linking products during sequencing library construction is provided, and the method comprises: a step of mixing an DNA endonuclease with the DNA-RNA-DNA:cDNA heterozygous chain and a DNA:cDNA double strand, wherein an enzyme cleavage site in the DNA-RNA-DNA:cDNA heterozygous chain is located in an RNA:cDNA pairing region, and an enzyme cleavage site in the DNA:cDNA double strand is located in a DNA:cDNA pairing region; and the DNA endonuclease can recognize the enzyme cleavage site in the DNA:cDNA double strand and perform cleavage.

In some implementations, the DNA endonuclease can effectively cleave a double-stranded DNA containing a sequence of the recognition site thereof, but has low activity for an RNA:cDNA heterozygous chain containing the sequence of the recognition site. In some implementations, the DNA-RNA-DNA:cDNA heterozygous chain is generated during construction of the sequencing library. In some implementations, the DNA in the DNA:cDNA double strand is a self-linking product formed by a 5' end linker and a 3' end linker used during RNA sequencing library construction. In some implementations, both the 5' end linker and the 3' end linker contain a partial sequence of the recognition site, and when the linker self-linking product is formed, a complete sequence of the recognition site is formed at the junction of the 5' end linker and the 3' end linker. In some implementations, the sequencing library is an RNA sequencing library. In some implementations, the 3' end linker and the 5' end linker respectively carry more than 30%, preferably more than 40%, and more preferably 50% of the sequence of the recognition site of the nucleic acid endonuclease, and when the self-linking product is formed, the complete sequence of the recognition site is formed at the junction of the 5' end linker and the 3' end linker. In some implementations, the DNA endonuclease may be selected from the DNA endonucleases listed in Table I.

According to another aspect of the present invention, a method for removing 5' and 3' linker self-linking products generated during RNA sequencing library construction, or a method for constructing the RNA sequencing library is provided, comprising the steps of:
(1) connecting a 3' end DNA linker to the 3' terminus of an RNA molecule;
(2) connecting a 5' end DNA linker to the 5' terminus of the RNA molecule connected with the 3' end linker;
(3) obtaining an extension product using an extension primer and an elongase based on the RNA molecule connected with the 3' end linker and the 5' end linker;
(4) performing enzyme cleavage treatment on the extension product using the DNA endonuclease to remove the linker self-linking products in a system; and
(5) performing PCR amplification using primers related to a sequence of the linker, the obtained amplification product being the sequencing library.

In some implementations, the 3' end linker and the 5' end linker each contain a partial sequence of a recognition site of the nucleic acid endonuclease, for example, more than 30%, preferably more than 40%, and more preferably 50% of the sequence of the recognition site, and when the linker self-linking product is formed, a complete sequence of the recognition site is formed at the junction of the 5' end linker and the 3' end linker. In some implementations, a content of the RNA molecule is ≥ 50 pg. In some implementations, the content of the RNA molecule is 50 pg-20 ng. In some implementations, the RNA molecule is a small RNA molecule. In some implementations, the small RNA molecule has a length of 15-200 nt. In some implementations, a cleavage efficiency of the DNA endonuclease for an RNA:cDNA heterozygous chain containing the sequence of the recognition site thereof is at most 1/10, at most 1/100, at most 1/1000, or at most 1/10000 of a cleavage efficiency for the corresponding DNA:cDNA double strand, and the DNA:cDNA double strand contains a cDNA of the same sequence and a DNA complementary thereto; and preferably, the DNA endonuclease is inactive, or has no detectable activity, for the RNA:cDNA heterozygous chain containing the sequence of the recognition site thereof. In some implementations, the ligation reaction of step (1) uses a truncated T4 RNA ligase 2 as a ligase. In some implementations, the elongase used in step (3) is a reverse transcriptase or Bst polymerase. In some implementations, the DNA endonuclease used in step (4) is a double-stranded DNA endonuclease. In some implementations, the DNA endonuclease may be selected from the DNA endonucleases listed in Table I. In some implementations, the DNA endonuclease is not Avail, AvrII, BanI, HaeIII, Hinfl, TaqI, or other enzymes that can cleave a DNA/RNA heterozygous chain under specific conditions.

By using the method for removing the 5' and 3' linker self-linking by-products generated in the construction process of the sequencing library according to an embodiment of the present invention, a sequencing library of small RNA molecules in a micro sample can be effectively constructed, and especially small RNA sequencing libraries of a plasma sample, a single cell sample, and other micro samples can be effectively constructed. Particularly, the library construction process provided in the present invention can optionally use a one-tube reaction process, further reducing an input amount in library construction, and reducing a deviation in the sequencing result caused by a purification process.

According to another aspect of the present invention, a kit for constructing a small RNA sequencing library is provided. According to an embodiment of the present invention, the kit comprises: an RNA 3' terminus connecting module, wherein the connecting module comprises a 3' end linker and is used for connecting the 3' end linker to the 3' terminus of an RNA; an RNA 5' terminus connecting module, wherein the connecting module comprises a 5' end linker and is used for connecting the 5' end linker to the 5' terminus of the RNA connected with the 3' end linker; an extension module, wherein the extension module comprises an elongase and is used for extending the RNA molecule connected with the 3' end linker and the 5' end linker; an enzyme cleavage module, wherein the enzyme cleavage module comprises a DNA endonuclease and is used for removing linker self-linking products in products of the extension module; and an amplification module, wherein the amplification module comprises an enzyme required for DNA amplification and is used for amplifying enzyme cleavage products, to obtain a final library. In some implementations, the extension module and the enzyme cleavage module can be integrated into one module, that is, an extension + enzyme cleavage module.

In some implementations, the RNA 3' terminus connecting module comprises a ligase; and preferably, the ligase is a truncated T4 RNA ligase 2 or a point mutant thereof. In some implementations, the RNA 5' terminus connecting module comprises a ligase, and preferably, the ligase is a T4 RNA ligase 1. In some implementations, the elongase is an MMLV reverse transcriptase and/or Taq enzyme. In some implementations, the DNA endonuclease is a double-stranded DNA endonuclease. In some implementations, the DNA endonuclease may be selected from the DNA endonucleases listed in Table I. In some implementations, the DNA endonuclease is not Avail, AvrII, BanI, HaeIII, Hinfl, TaqI, or other enzymes that can cleave a DNA/RNA heterozygous chain under specific conditions.

By using the kit for constructing the sequencing library of the small RNA molecules according to an implementation of the present invention, a micro sample of small RNAs can be used to construct a sequencing library with low sample loss and intact sequence information, and the kit can be applied to library construction of small RNA samples extracted from plasma and small RNA samples in single cells.

The RNA samples can be obtained by any means, and sources of the samples include but are not limited to organisms, organs, tissues, cells, organelles, etc.

According to another aspect of the invention, use of the method according to the present invention or the kit according to the present invention in construction of an RNA sequencing library is provided. In some implementations, the RNA sequencing library is selected from the group of: a plasma small RNA sequencing library, a CLIP library, an RIP library, an MeRIP library, and a GRO library.

According to yet another aspect of the present invention, a method for determining sequence information of a small RNA molecule is provided, and according to an implementation of the present invention, the method comprises: constructing a sequencing library based on a small RNA sample by the method according to the present invention; sequencing the sequencing library to obtain a sequencing result; and determining the sequence information of the small RNA molecule based on the sequencing result.

According to yet another aspect of the present invention, a system for determining sequence information of a small RNA molecule is provided. According to an implementation of the present invention, the system comprises: a sequencing library construction device, wherein the sequencing library construction device is the kit according to the present invention; a sequencing device, wherein the sequencing device is used for sequencing a sequencing library constructed for the sample by the kit to obtain a sequencing result of the sample; and an analysis device, for analyzing the sequencing result of the sample to obtain the sequence information of the small RNA molecule.

By using the system for determining the sequence information of the small RNA molecule according to an implementation of the present invention, sequence information of small RNAs in a micro sample can be sensitively, accurately, and efficiently determined.

Another objective of the present invention is to provide a method for simultaneous sequencing of DNA and RNA that is simple to operate, highly sensitive, and suitable for micro samples and plasma samples. The method can avoid a problem of confusing a single-stranded DNA molecule and an RNA molecule in the prior art, additionally has low preference for the 5' and 3' termini of nucleic acids, and can retain sequence information of termini of the DNA and RNA molecules.

To achieve the foregoing objective, the present invention adopts the following technical solutions:
According to one aspect of the present invention, a method for sequencing DNA and RNA molecules in the same sample is provided, and the method comprises the steps of:
(1) connecting a specific tag linker to the 3' terminus of an RNA molecule in a mixed sample of DNA and RNA molecules;
(2) obtaining a single-stranded DNA molecule using a reverse transcription primer based on the RNA molecule connected with the tag linker;
(3) constructing a library for all DNA molecules in the system; and
(4) sequencing the library, and using a sequence of the specific tag linker to distinguish the RNA molecules in the mixed sample.

By using the method for simultaneous sequencing of DNA and RNA molecules in the same sample according to an embodiment of the present invention, a sequencing library of the DNA and RNA molecules in the same sample can be effectively constructed, and especially a sequencing library of a cfDNA/cfRNA sample in plasma can be effectively constructed. Connecting the specific tag linker to the 3' terminus of the RNA using a truncated T4 RNA ligase 2 can avoid a case that the tag linker is connected to the 3' end of a single-stranded DNA, and reduce the deviation of sequencing data. In addition, after the RNA is reversed into a single-stranded DNA, the subsequent library construction process can choose to use a one-tube reaction process, which reduces a starting amount of library construction and can be efficiently and sensitively applied to the high-throughput sequencing technology, and based on data analysis on a sequencing result, sequence information of the DNA and RNA can be effectively obtained.

The mixed sample of the DNA and RNA can be obtained by any means, and sources of the nucleic acid sample include but are not limited to organisms, organs, tissues, cells, organelles, etc. In some embodiments, the sample contains a cell-free DNA and cell-free RNA.

According to another aspect of the present invention, the present invention provides a kit for constructing a sequencing library of DNA and RNA molecules in the same sample. According to an embodiment of the present invention, the kit comprises: an RNA terminus connecting module, wherein the connecting module comprises a specific tag linker used for connecting to the 3' terminus of a single-stranded RNA; a reverse transcription module, wherein the reverse transcription module is connected with the RNA terminus connecting module, and the reverse transcription module comprises a reverse transcription primer and a reverse transcriptase and is used for obtaining a single-stranded DNA molecule through reverse transcription based on the RNA molecule connected with the specific tag linker in the previous step; and a DNA library construction module, wherein the DNA library construction module is connected with the reverse transcription module and is used for constructing the DNA molecule in a system into the sequencing library.

According to another aspect of the present invention, a kit for constructing a sequencing library of DNA and RNA molecules in the same sample according to an embodiment of the present invention is provided, wherein a micro mixed sample of single-stranded DNA and RNA can be used to construct a sequencing library, and the DNA and RNA molecules can be distinguished, with low sample loss and intact sequence information, and the kit can be applied to library construction of a mixed sample of cfDNA and cfRNA extracted from plasma.

According to yet another aspect of the present invention, a method for determining sequence information of DNA and RNA molecules in a mixed sample of the DNA and RNA molecules is provided, and according to an embodiment of the present invention, the method comprises: constructing a sequencing library based on the mixed sample of the DNA and RNA molecules by the method according to the present invention; sequencing the sequencing library to obtain a sequencing result; and determining the sequence information of the DNA and RNA molecules in the mixed sample based on the sequencing result.

According to yet another aspect of the present invention, a system for determining sequence information of DNA and RNA molecules is provided. According to an embodiment of the present invention, the system comprises: a sequencing library construction device, wherein the sequencing library construction device may be the kit according to the present invention; a sequencing device, wherein the sequencing device is connected with the sequencing library construction device to sequence a sequencing library of the sample and obtain a sequencing result of the sample; and an analysis device, for analyzing the sequencing result of the sample to obtain the sequence information of the DNA and RNA molecules.

By using the system for determining the sequence information of the DNA and RNA molecules according to the embodiment of the present invention, sequence information of DNA and RNA molecules in a micro sample can be sensitively, accurately, and efficiently determined, and physical association information of the two can be retained.

According to another aspect of the present invention, a method for determining sequence information of a DNA target region being transcribed and a synthesized RNA molecule in a transcription complex is provided, and according to an embodiment of the present invention, the method comprises: randomly fragmenting chromatin to obtain a chromatin sample of 200 bp-500 bp in length; performing co-immunoprecipitation on the chromatin sample using an antibody for an RNA polymerase, and obtaining a double-stranded DNA and RNA sample in the region being transcribed after degrading proteins using proteinase K; constructing a sequencing library for the double-stranded DNA and RNA sample by the method according to the present invention; sequencing the sequencing library; and determining, based on the sequencing result, the sequence information of the DNA and RNA molecules in the region being transcribed.

Additional aspects and advantages of the present invention will be partially provided in the following description, and a part thereof will be apparent from the following description, or learned by practice of the present invention.

### Embodiments

Embodiments of the present invention are described in detail below, and examples of the embodiments are illustrated in the drawings. Those skilled in the art will understand that the embodiments described below with reference to the drawings are exemplary and are only used for explaining the present invention, but cannot be understood as limitations to the present invention. Where specific techniques or conditions are not indicated in the embodiments, the techniques or conditions described in the literature in the art or the product specifications shall be followed. The reagents or instruments used without the manufacturer being specified are all conventional products commercially available, such as from NEB and other companies.

### Embodiment 1: Removing linker self-linking products based on DNA single and double strands and an endonuclease

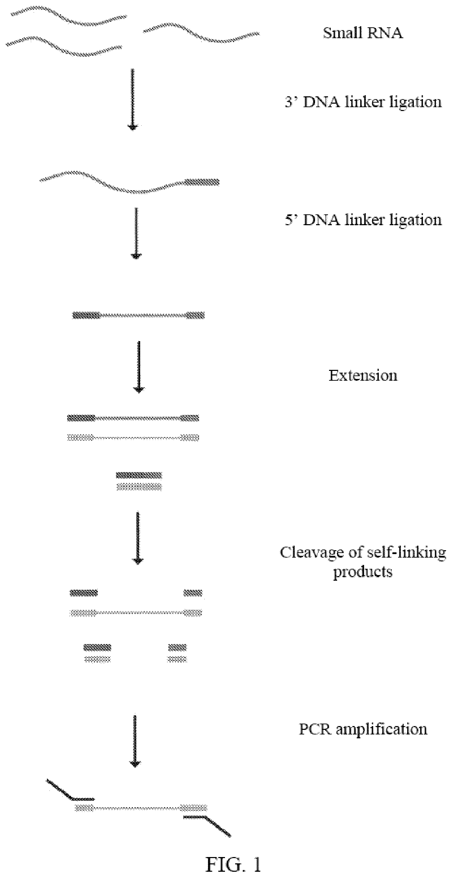

According to one aspect of the present invention, the present invention provides a method for removing self-linking linkers during RNA library construction and a method for constructing a small RNA library. Referring to FIG. 1, according to the embodiment of the present invention, the method may include the following steps:
1. A small RNA sample used for library construction is first quantified using Qubit 4.0, and a Qubit microRNA Assay Kit is used for quantification. A small RNA part for library construction may have a starting amount of 50 pg-20 ng.
2. A small RNA molecule is connected with a 3' end DNA linker.
   2-1 The small RNA molecule sample is mixed with the 3' end DNA linker for a reaction at 70°C for 2 minutes, and then a ligase is used to perform ligation in a ligation reaction system.
   2-2 The sequence of the 3' end DNA linker is 5'-rAPP-CGCAAGTCGGAGGCCAAG-3'ddC (SEQ ID NO: 1), the 5' end of the linker sequence has an adenylation modification, and the 3' end has a dideoxy modification, thereby preventing self-linking of the linker during the ligation reaction. At the same time, the 5' end of the linker has a half of the recognition sequence of a BssHII enzyme, which is used for the subsequent linker self-linking removal process.
   2-3 The ligase used in this ligation reaction is a truncated T4 RNA ligase 2 and various point mutants thereof, the enzyme can only connect an adenylated single-stranded DNA or RNA linker to the 3' terminus of the RNA molecule, thereby avoiding self-linking between the small RNA molecules.
   2-4 The ligation reaction system includes: a thermal denaturation product obtained in 2-1, the 3' end DNA linker, the truncated T4 RNA ligase 2, a ligase reaction buffer, PEG8000, and an RNA enzyme inhibitor.
   2-5 Reaction conditions are at 16°C overnight, reacting at 25°C for 2 hours, or reacting at 37°C for 30 minutes.
3. The small RNA molecule connected with the 3' end linker is connected with a 5' end DNA linker.
   3-1 The small RNA molecule sample connected with the 3' end linker is mixed with the 5' end DNA linker for a reaction at 70°C for 2 minutes, and then ligation is performed in the ligation reaction system.
   3-2 The sequence of the 5' end DNA linker is 5'-GCTACGATCCGACTTNNNNGCG-3' (SEQ ID NO: 2), and the 3' end of the linker has the other half of the recognition sequence of a PauI enzyme, which is used for the subsequent linker self-linking removal process.
   3-3 The ligation reaction system includes: a ligation product obtained in 2-5, the 5' end DNA linker, PEG8000, a ligase buffer, and a T4 RNA ligase 1.
   3-4 Reaction conditions are at 16°C overnight or reacting at 25°C for 2 hours.
4. Extension reaction.
   4-1 Extension is performed using a ligation product obtained in 3-4 as a template and using an extension primer designed according to the known sequence of the 3' end DNA linker to obtain an extension product.
   4-2 The sequence of the extension primer is: 5'-TTGTCTTCCTAAGACCGCTTGGCCTCCGACTTGCG-3' (SEQ ID NO: 6).
   4-3 The extension reaction system includes: the ligation product obtained in 3-4, the extension primer, an extension reaction buffer, and a reverse transcriptase.
   4-4 Reaction conditions: reacting at 42°C for 30 minutes.
5. Enzyme cleavage treatment.
   5-1 Enzyme cleavage treatment is performed on the extension product obtained in 4-4 to remove self-linking linkers.
   5-2 Enzymes used are RNase Hand BssHII.
   5-3 Reaction conditions are reacting at 37°C for 30 minutes.
6. Second-strand synthesis reaction.
   6-1 A second-strand synthesis product is synthesized using a product after the enzyme cleavage treatment obtained in 5-3 as a template and using a second-strand synthesis primer designed according to a known sequence of the product obtained by extension.
   6-2 The sequence of the second-strand synthesis primer is: 5'-CAACTCCTTGGCTCACAGAACGACATGGCTACGATCCGACTT-3' (SEQ ID NO: 10).
   6-3 The reaction system includes: the product after the enzyme cleavage, the second-strand synthesis primer, a DNA polymerase buffer, and a DNA polymerase.
   6-4 Reaction conditions are: denaturing at 98°C for 2 minutes; at 60°C for 30 seconds; reacting at 72°C for 5 minutes.
7. PCR amplification.
   7-1 PCR amplification is performed using the second-strand synthesis product obtained in 6-4 as a template and using forward and reverse primers designed according to known sequences of the extension primer and the second-strand synthesis primer, and a certain number of random bases can be introduced into the forward and reverse primers for distinguishing different samples in high-throughput sequencing.
   7-2 The forward and reverse PCR primers are synthesized.
   Sequence of the forward primer: 5'-GCATGGCGACCTTATCAGTTGTCTTCCTAAGACCGCTTGG-3' (SEQ ID NO: 11).
   Sequence of the reverse primer: 5'-Pho-CTCTCAGTACGTCAGCAGTTNNNNNNNNNNCAACTCCTTGGCTCACAGAAC-3' (SEQ ID NO: 12).
   There are 10 random bases used for distinguishing different samples in the high-throughput sequencing.
   7-3 The reaction system includes: the second-strand synthesis product obtained in 6-4, the forward and reverse PCR primers, a high-fidelity DNA polymerase, and the DNA polymerase buffer.
   7-4 Reaction conditions: denaturing at 98°C for 2 minutes; and at 98°C for 15 seconds, at 60°C for 30 seconds, and at 72°C for 30 seconds, and this process is repeated for 15-18 cycles during the reaction; and then reacting at 72°C for 5 minutes.
8. PCR products are purified using a magnetic beads method to obtain target products, and the target products are recovered to obtain the library.

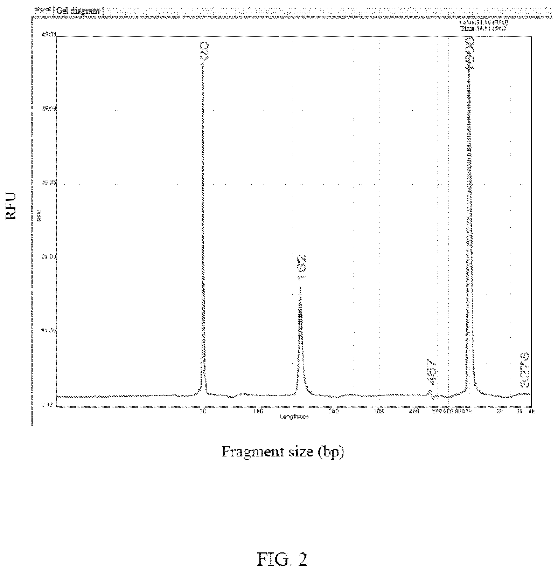

FIG. 2 shows a quality inspection chart of fragment sizes of the constructed small RNA library.

It can be seen from FIG. 2 that the sizes of the obtained library fragments are consistent with the length sizes of the small RNA fragments, and there are no obvious linker residues at the same time, indicating that in the present embodiment, a qualified small RNA mixed library is successfully obtained, and removal of the self-linking products is achieved at the same time.

### Example 2: Removing linker self-linking products based on a DNA double strand and an RNA: DNA heterozygous chain

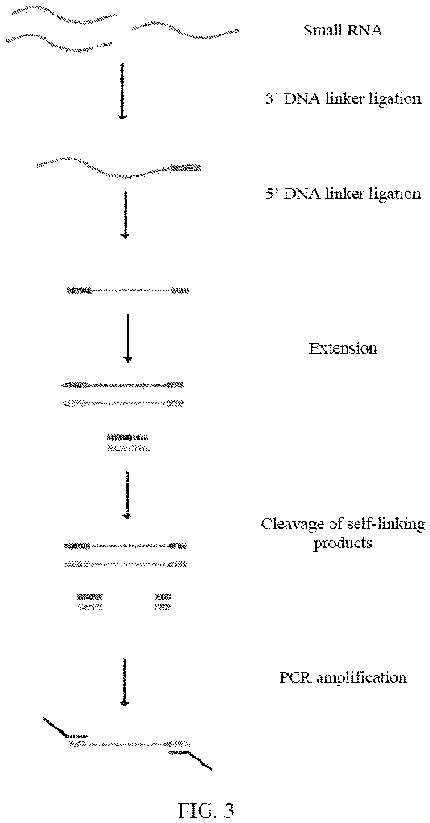

According to one aspect of the present invention, the present invention provides a method for removing self-linking linkers during RNA library construction and a method for constructing a small RNA library. Referring to FIG. 3, according to the embodiment of the present invention, the method may include the following steps:
1. A small RNA sample used for library construction is first quantified using Qubit 4.0, and a Qubit microRNA Assay Kit is used for quantification. A small RNA part for library construction may have a starting amount of 50 pg-20 ng.
2. A small RNA molecule is connected with a 3' end DNA linker.
   2-1 The small RNA molecule sample is mixed with the 3' end DNA linker for a reaction at 70°C for 2 minutes, and then a ligase is used to perform ligation in a ligation reaction system.
   2-2 The sequence of the 3' end DNA linker is 5'-rAPP-CGCAAGTCGGAGGCCAAG-3'ddC (SEQ ID NO: 1), the 5' end of the linker sequence has an adenylation modification, and the 3' end has a dideoxy modification, thereby preventing self-linking of the linker during the ligation reaction. At the same time, the 5' end of the linker has a half of the recognition sequence of a PauI enzyme, which is used for the subsequent linker self-linking removal process.
   2-3 The ligase used in this ligation reaction is a truncated T4 RNA ligase 2 and various point mutants thereof, the enzyme can only connect an adenylated single-stranded DNA or RNA linker to the 3' terminus of the RNA molecule, thereby avoiding self-linking between the small RNA molecules.
   2-4 The ligation reaction system includes: a thermal denaturation product obtained in 2-1, the 3' end DNA linker, the truncated T4 RNA ligase 2, a ligase reaction buffer, PEG8000, and an RNA enzyme inhibitor.
   2-5 Reaction conditions are at 16°C overnight, reacting at 25°C for 2 hours, or reacting at 37°C for 30 minutes.
3. The small RNA molecule connected with the 3' end linker is connected with a 5' end DNA linker.
   3-1 The small RNA molecule sample connected with the 3' end linker is mixed with the 5' end DNA linker for a reaction at 70°C for 2 minutes, and then ligation is performed in the ligation reaction system.
   3-2 The sequence of the 5' end DNA linker is 5'-GCTACGATCCGACTTNNNNGCG-3' (SEQ ID NO: 2), and the 3' end of the linker has the other half of the recognition sequence of a PauI enzyme, which is used for the subsequent linker self-linking removal process.
   3-3 The ligation reaction system includes: a ligation product obtained in 2-5, the 5' end DNA linker, PEG8000, a ligase buffer, and a T4 RNA ligase 1.
   3-4 Reaction conditions are at 16°C overnight or reacting at 25°C for 2 hours.
4. Extension reaction.
   4-1 Extension is performed using a ligation product obtained in 3-4 as a template and using an extension primer designed according to the known sequence of the 3' end linker, to obtain an extension product.
   4-2 The sequence of the extension primer is: 5'-TTGTCTTCCTAAGACCGCTTGGCCTCCGACTTGCG-3' (SEQ ID NO: 6).
   4-3 The extension reaction system includes: the ligation product obtained in 3-4, the extension primer, an extension reaction buffer, a reverse transcriptase, and a DNA polymerase.
   4-4 Reaction conditions: reacting at 42°C for 30 minutes.
5. Enzyme cleavage treatment.
   5-1 Enzyme cleavage treatment is performed on the extension product obtained in 4-4, to remove self-linking linkers.
   5-2 The DNA endonuclease used is PauI.
   5-3 Reaction conditions are reacting at 37°C for 30 minutes.
6. Second-strand synthesis reaction.
   6-1 A second-strand synthesis product is synthesized using a product after the enzyme cleavage treatment obtained in 5-3 as a template and using a second-strand synthesis primer designed according to a known sequence of the product obtained by extension.
   6-2 The sequence of the second-strand synthesis primer is: 5'-CAACTCCTTGGCTCACAGAACGACATGGCTACGATCCGACTT-3' (SEQ ID NO: 10).
   6-3 The reaction system includes: the product after the enzyme cleavage, the second-strand synthesis primer, a DNA polymerase buffer, and a DNA polymerase.
   6-4 Reaction conditions are: denaturing at 98°C for 2 minutes; at 60°C for 30 seconds; reacting at 72°C for 5 minutes.
7. PCR amplification.
   7-1 PCR amplification is performed using the second-strand synthesis product obtained in 6-4 as a template and using forward and reverse primers designed according to known sequences of the extension primer and the second-strand synthesis primer, and a certain number of random bases can be introduced into the forward and reverse primers for distinguishing different samples in high-throughput sequencing.
   7-2 The forward and reverse PCR primers are synthesized.
   Sequence of the forward primer: 5'-GCATGGCGACCTTATCAGTTGTCTTCCTAAGACCGCTTGG-3' (SEQ ID NO: 11).
   Sequence of the reverse primer:
      5'-Pho-CTCTCAGTACGTCAGCAGTTNNNNNNNNNNCAACTCCTTGGCTCACAGAAC-3' (SEQ ID NO: 12).
   There are 10 random bases used for distinguishing different samples in the high-throughput sequencing.
   7-3 The reaction system includes: the second-strand synthesis product obtained in 6-4, the forward and reverse PCR primers, a high-fidelity DNA polymerase, and the DNA polymerase buffer.
   7-4 Reaction conditions: denaturing at 98°C for 2 minutes; and at 98°C for 15 seconds, at 60°C for 30 seconds, and at 72°C for 30 seconds, and this process is repeated for 15-18 cycles during the reaction; and then reacting at 72°C for 5 minutes.
8. PCR products are purified using a magnetic beads method to obtain target products, and the target products are recovered to obtain the library.

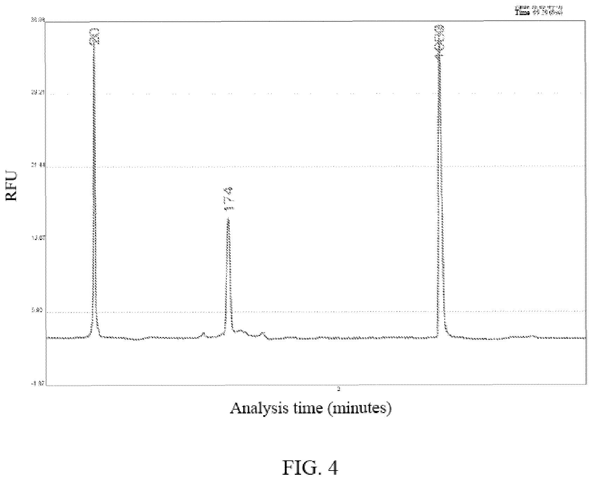

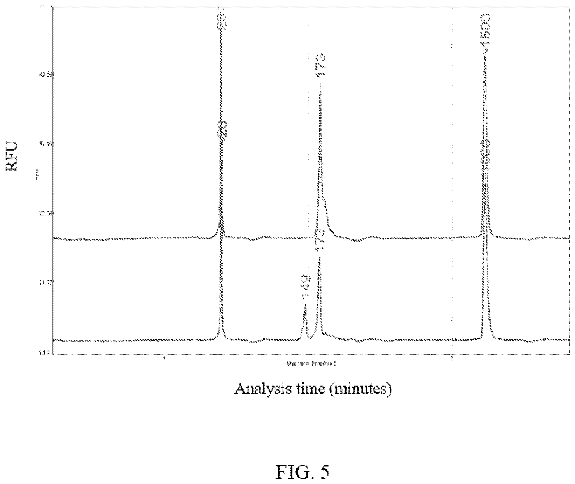

FIG. 4 and FIG. 5 show a quality inspection chart of fragment sizes of the constructed small RNA library. The upper band in FIG. 5 is a small RNA library using the present embodiment, and the lower band is a small RNA library using the same process as the present embodiment, but with a step of endonuclease treatment removed;

It can be seen from FIG. 4 and FIG. 5 that, the sizes of the obtained library fragments are consistent with the length sizes of the small RNA fragments, and there are no obvious linker residues at the same time, indicating that in the present embodiment, a qualified small RNA mixed library is successfully obtained. At the same time, compared with a library treated without the endonuclease, the library treated with the enzyme has a significantly lower proportion of linker self-linking product residues, indicating that the removal of the self-linking products is achieved in a heterozygous chain manner.

### Example 3: DNA and RNA library co-construction and sequencing

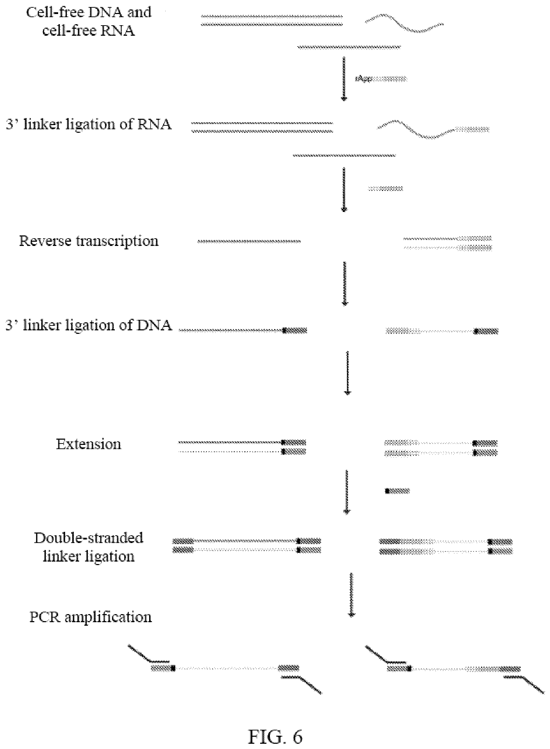

According to one aspect of the present invention, the present invention provides a method for simultaneous sequencing of DNA and RNA molecules in the same sample. Referring to FIG. 6, according to the embodiment of the present invention, the method may include the following steps:
1. A DNA and RNA sample used for library construction is first quantified using Qubit 4.0, and a 1* Qubit dsDNA HS Assay Kit and a Qubit microRNA Assay Kit are used for quantification, respectively. A DNA part for library construction may have a starting amount of 0.5-20 ng, and an RNA part for the library construction may have a starting amount of 100 pg-10 ng.
2. An RNA molecule is connected with a specific tag linker.
   2-1 The sample of DNA and RNA molecules is mixed with the specific tag linker for a reaction at 70°C for 2 minutes, and then a ligase is used to perform ligation in a ligation reaction system.
   2-2 The sequence of the specific tag linker is 5'-rAPP-(N)ₙTGATGCGTAGCCTTAA-3'ddC (SEQ ID NO: 3), the 5' end of the linker sequence has an adenylation modification, and the 3' end has a dideoxy modification, thereby preventing self-linking of the linker and self-linking between the DNA and RNA molecules during the ligation reaction. At the same time, a random sequence of n bases is introduced at the 5' end of the linker to correct PCR amplification deviations that may be caused when constructing a library with a low starting amount, and n is a positive integer from 1 to 15.
   2-3 The ligase used in this ligation reaction is a truncated T4 RNA ligase 2 and various point mutants thereof, and the enzyme can only connect an adenylated single-stranded DNA or RNA linker to the 3' terminus of the RNA molecule, but not to the 3' terminus of the DNA molecule, thereby achieving a specific connection with the RNA molecule.
   2-4 The ligation reaction system includes: a thermal denaturation product obtained in 2-1, the specific tag linker, the truncated T4 RNA ligase 2, a ligase reaction buffer, PEG8000, and an RNA enzyme inhibitor.
   2-5 Reaction conditions are at 16°C overnight, reacting at 25°C for 2 hours, or reacting at 37°C for 30 minutes.
3. A single-stranded DNA molecule is obtained using a reverse transcription primer based on the RNA molecule with the tag linker.
   3-1 Reverse transcription is performed using a ligation product obtained in the previous step as a template and using a reverse transcription primer designed according to the known sequence of the tag linker to obtain a single-stranded DNA molecule.
   3-2 The sequence of the reverse transcription primer is 5'-GTTAAGGCTACGCATCA-3' (SEQ ID NO: 4).
   3-3 A reverse transcription reaction system includes the ligation product obtained in 2-5, the reverse transcription primer, a reverse transcriptase buffer, a reverse transcriptase, and the RNA enzyme inhibitor.
   3-4 Reaction conditions are determined based on the optimal reaction temperature of the reverse transcriptase.
4. A reverse transcription product is purified using a magnetic beads method.
5. Dephosphorylation treatment and high-temperature denaturation.
   A purified product obtained in 4 is dephosphorylated, the reaction system includes an alkaline phosphatase and a reaction buffer, and a reaction is performed at 37°C for 30 minutes, and then at 95°C for 5 minutes to denature the DNA into a single strand at a high temperature.
6. A single-stranded linker is connected with the single-stranded DNA product obtained in 5, and this connection is a connection between two single-stranded DNA molecules.
   6-1 The sequence of the single-stranded linker is 5'-Pho-(N)ₙAAGTCGGATCGTAGCCATG-3' ddC (SEQ ID NO: 5), and the linker sequence has a phosphorylation modification at the 5' end and a dideoxy modification at the 3' end, thereby preventing self-linking of the linker and self-linking between DNA molecules during the ligation reactions. At the same time, a random sequence of n bases is introduced at the 5' end of the linker, to correct PCR amplification deviations that may be caused when constructing a library with a low starting amount, and n is a positive integer from 1 to 15.
   6-2 The ligation reaction system includes: the single-stranded DNA product obtained in 5, the single-stranded linker, PEG8000, a ligase buffer, and a T4 RNA ligase.
   6-3 Reaction conditions are at 16°C overnight or reacting at 30°C for 2 hours.
7. Extension reaction. Extension is performed using the single-stranded DNA product obtained in 5 or a ligation product obtained in 6-3 as a template and using a primer designed according to the known sequence of the single-stranded linker, to obtain a double-stranded DNA product.
   7-1 The sequence of the primer is: 5'-CAACTCCTTGGCTCACAGAACGACATGGCTACGATCCGACTT-3' (SEQ ID NO: 7).
   7-2 The reaction system includes: the single-stranded DNA product obtained in 5 or the single-stranded DNA ligation product obtained in 6-3, the primer, a DNA polymerase buffer, and a DNA polymerase.
   7-3 Reaction conditions: at 95°C for 2 minutes, at 45°C for 30 seconds, and at 72°C for 5 minutes.
8. Double-end linker ligation This reaction step is an intermolecular connection between the double-stranded DNA product obtained in 7-3 and a double-stranded DNA linker.
   8-1 The sequence of the double-stranded DNA linker is: F sequence: 5'-Pho-GAAGTCGGAGGCCAAGCGGTCTTAGGAAGACAA-3' (SEQ ID NO: 8).
   R sequence: 5'-CAACTCCTTGGCTCACAGAACGACATGGCTACGATCCGACTTCT-3' (SEQ ID NO: 9).
   The R sequence is reversely complementary to the F sequence, and in particular, there is a protruding base T at the end of the R sequence.
   8-2 The ligation reaction system includes: the double-stranded DNA product obtained in 7-3, the double-stranded DNA linker, a T4 DNA ligase, and a T4 DNA ligase buffer.
   8-3 Reaction conditions are reacting at 20°C for 30 minutes.
9. PCR amplification. PCR amplification is performed using a ligation product obtained in 8-3 as a template and using forward and reverse primers designed according to known sequences of the 5' end of the R sequence and the 3' end of the F sequence in the sequence of the double-stranded DNA linker, and a certain number of random bases can be introduced into the forward and reverse primers for distinguishing different samples in high-throughput sequencing.
   9-1 The forward and reverse PCR primers are synthesized.
   Sequence of the forward primer: 5'-GCATGGCGACCTTATCAGTTGTCTTCCTAAGACCGCTTGG-3' (SEQ NO: 11).
   Sequence of the reverse primer: 5'-Pho-CTCTCAGTACGTCAGCAGTTNNNNNNNNNNCAACTCCTTGGCTCACAGAAC-3' (SEQ NO: 12).
   There are 10 random bases used for distinguishing different samples in the high-throughput sequencing.
   9-2 The reaction system includes: a ligation product in the previous step, the forward and reverse PCR primers, a high-fidelity DNA polymerase, and the DNA polymerase buffer.
   9-3 Reaction conditions: denaturing at 98°C for 2 minutes; and at 98°C for 15 seconds, at 60°C for 30 seconds, and at 72°C for 30 seconds, and this process is repeated for 8-15 cycles during the reaction; and then reacting at 72°C for 5 minutes.
10. PCR products are purified using a magnetic beads method to obtain target products, and the target products are recovered to obtain the library.

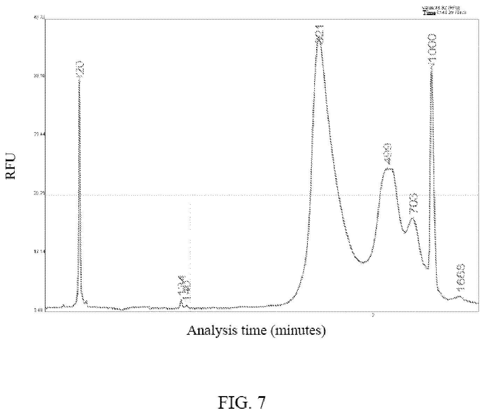

FIG. 7 shows a quality inspection chart of fragment sizes of a DNA/RNA mixed library. It can be seen from the quality inspection chart that the sizes of the obtained library fragments are consistent with the length sizes of cfDNA/cfRNA fragments, and there are no obvious linker residues at the same time, indicating that in the present embodiment, a qualified DNA/RNA mixed library is successfully obtained.

The foregoing content is a further detailed description of the present invention in combination with specific implementations, and cannot be construed that the specific implementations of the present invention are limited to these descriptions. Those skilled in the art can understand that various changes, modifications, substitutions, and variations can be made to these embodiments without departing from the principle and purpose of the present invention, and the scope of the present invention is defined by the claims and their equivalents.

| SEQ NO | Name | Sequence | Notes |
|---|---|---|---|
| 1 | 3' end DNA linker | CGCAAGTCGGAGGCCAAG | |
| 2 | 5' end DNA linker | GCTACGATCCGACTTNNNNGCG | |
| 3 | Specific tag linker | (N)nTGATGCGTAGCCTTAA | n may be a positive integer from 1 to 15 |
| 4 | Reverse transcription primer | GTTAAGGCTACGCATCA | |
| 5 | Single-stranded linker | (N)nAAGTCGGATCGTAGCCATG | n may be a positive integer from 1 to 15 |
| 6 | Extension primer | TTGTCTTCCTAAGACCGCTTGGCCTCCGA CTTGCG | |
| 7 | Extension primer | CAACTCCTTGGCTCACAGAACGACATGG CTACGATCCGACTT | |
| 8 | F sequence of a double-stranded linker | GAAGTCGGAGGCCAAGCGGTCTTAGGAA GACAA | |
| 9 | R sequence of the double-stranded linker | CAACTCCTTGGCTCACAGAACGACATGG CTACGATCCGACTTCT | |
| 10 | Second-strand synthesis primer | CAACTCCTTGGCTCACAGAACGACATGG CTACGATCCGACTT | |
| 11 | Forward primer | GCATGGCGACCTTATCAGNNNNNNNNNN | |
| | | TTGTCTTCCTAAGACCGCTTGG | |
| 12 | Reverse primer | CTCTCAGTACGTCAGCAGTTNNNNNNNN NNCAACTCCTTGGCTCACAGAAC | |

## Claims

1. A method for constructing a sequencing library for an RNA molecule in a sample, comprising the steps of:
(1) connecting a 3' end linker to the 3' terminus of the RNA molecule in the sample;
(2) connecting a 5' end linker to the 5' terminus of the RNA molecule in the sample connected with the 3' end linker;
(3) obtaining an extension product using an elongase and an extension primer based on the RNA molecule in the sample connected with the 3' end linker and the 5' end linker;
(4) treating the extension product using a DNA endonuclease and an enzyme capable of specifically hydrolyzing an RNA in a DNA-RNA heterozygous chain; and
(5) performing PCR amplification on the extension product, to obtain the sequencing library, wherein both the 5' end linker and the 3' end linker contain a partial sequence of a recognition site of the DNA endonuclease, and when a self-linking product of the 5' end linker and the 3' end linker is formed, a complete sequence of the recognition site is formed at the junction; and preferably, the enzyme capable of specifically hydrolyzing the RNA in the DNA-RNA heterozygous chain is RNase H.

2. The method according to Claim 1, wherein the ligation reaction of step (1) uses a truncated T4 RNA ligase 2 or a point mutant thereof, and/or the ligation reaction of step (2) uses a T4 RNA ligase 1.

3. The method according to Claim 1 or 2, wherein the elongase used in step (3) is a reverse transcriptase and/or Bst polymerase; and preferably, the reverse transcriptase is an MMLV reverse transcriptase.

4. The method according to any one of Claims 1 to 3, wherein the RNA molecule in the sample is a small RNA molecule; and preferably, the small RNA molecule has a length of 15-200 nt.

5. The method according to any one of Claims 1 to 4, wherein a content of the RNA molecules in the sample is ≤ 100 pg, ≤ 200 pg, ≤ 500 pg, ≤ 1 ng, ≤ 2 ng, ≤ 5 ng, ≤ 10 ng, or ≤ 20 ng.

6. The method according to any one of Claims 1 to 5, wherein the content of the RNA molecules in the sample is ≥ 50 pg; and preferably, the content of the RNA molecules in the sample is 50 pg-20 ng.

7. A method for processing a sample containing a DNA double strand and a DNA single strand, wherein the method comprises adding a DNA endonuclease to the sample, and
a DNA:DNA pairing region of the DNA double strand contains a sequence of a recognition site of the DNA endonuclease;
optionally, the DNA single strand and the DNA double strand are generated during construction of an RNA sequencing library;
optionally, the DNA double strand comprises a self-linking product formed by a 5' end linker and a 3' end linker used during RNA sequencing library construction; and
optionally, when the self-linking product is formed, the sequence of the recognition site of the DNA endonuclease is formed at the junction of the 5' end linker and the 3' end linker.

8. The method according to any one of Claims 1 to 7, wherein the method can efficiently remove the self-linking product formed by the 5' end linker and the 3' end linker in a system; and preferably, the method can remove at least 50%, at least 60%, at least 70%, at least 80%, at least 90%, at least 95%, at least 98%, at least 99%, at least 99.5%, at least 99.8%, or at least 99.9% of the self-linking products in the system.

9. The method according to any one of Claims 1 to 8, wherein the 5' end of the 3' end linker carries at least one, at least two, at least three, or at least four consecutive nucleotides of the 3' end of the recognition site of the DNA endonuclease; optionally, the 5' end of the 3' end linker has an adenylation modification, and the 3' end has a dideoxy modification; and optionally, the 3' end linker is a DNA linker.

10. The method according to any one of Claims 1 to 9, wherein the 3' end of the 5' end linker carries at least one, at least two, at least three, or at least four consecutive nucleotides of the 5' end of the recognition site of the DNA endonuclease; and optionally, the 5' end linker is a DNA linker.

11. The method according to any one of Claims 1 to 10, wherein neither the 5' end linker nor the 3' end linker comprises the complete sequence of the recognition site of the DNA endonuclease.

12. The method according to any one of Claims 1 to 11, wherein the DNA endonuclease can effectively cleave the DNA double strand containing the sequence of the recognition site thereof, but has low activity for a DNA single strand containing the sequence of the recognition site.

13. The method according to Claim 12, wherein a cleavage efficiency of the DNA endonuclease for the DNA single strand containing the sequence of the recognition site thereof is at most 1/10, at most 1/100, at most 1/1000, or at most 1/10000 of a cleavage efficiency for a corresponding DNA:DNA double strand, the DNA:DNA double strand contains the DNA single strand of the same sequence and a DNA single strand complementary thereto; and preferably, the DNA endonuclease is inactive, or has no detectable activity, for the DNA single strand containing the sequence of the recognition site thereof.

14. The method according to any one of Claims 1 to 13, wherein the DNA endonuclease is a double-stranded DNA endonuclease; and preferably, the DNA endonuclease is selected from the group of: AatII, BamHI, BsaBI, BsrFI, DraI, HphI, NdeI, Paul, PvuII, SwaI, Acc65I, BanI, BsaHI, BsrGI, DraIII, Hpy188I, NgoMI, RsaI, TaqI, AccI, BanII, BsaI, BsrI, DrdI, Hpy188III, NheI, RsrII, TfiI, AciI, BbsI, BsaJi, BssHI, BssHII, EaeI, Hpy99I, NlaIII, SacI, TliI, AclI, BbvCI, BsaWI, BssKI, EagI, HpyCH4III, NlaIV, SacII, TseI, AcuI, BbvI, BsaXI, BssSI, EarI, HpyCH4IV, NotI, SalI, Tsp45I, AfeI, BccI, BseRI, BstAPI, EciI, HpyCH4V, NruI, SapI, Tsp509I, AflII, BceAI, BseYI, BstBI, EcoNI, KasI, NsiI, Sau3AI, TspRI, AflIII, BcgI, BsgI, BstEII, EcoO109I, KpnI, NspI, Sau96I, Tth111I, AgeI, BciVI, BsiEI, BstF5I, EcoRI, MboI, PacI, SbfI, XbaI, AhdI, Bell, BsiHKAI, BstNI, EcoRV, MboII, PaeR7I, ScaI, XcmI, AleI, BfaI, BsiWI, BstUI, FatI, MfeI, PciI, ScrFI, XhoI, AluI, BfrBI, BsiI, BstXI, Paul, MluI, PflFI, SexAI, XmaI, AlwI, BfuAI, BsmAI, BstYI, Fnu4HI, MlyI, PflMI, SfaNI, XmnI, AlwNI, BfuCI, BsmBI, BstZ17I, FseI, MmeI, PhoI, SfcI, ZraI, ApaI, BglI, BsmFI, Bsu36I, FspI, MnlI, PleI, SfoI, ApaLI, BglII, BsmI, BtgI, HaeII, MscI, PmeI, SgrAI, Nb.BbvCI, ApeKI, BlpI, BsoBI, BtgZI, HaeIII, MseI, PmlI, SmaI, Nt.BbvCI, ApoI, Bme1580I, Bsp1286I, BtsI, HgaI, MsiI, PpuMI, SmlI, Nb.BsmI, AscI, BmgBI, BspCNI, Cac8I, HhaI, MspA1I, PshAI, SnaBI, Nt.BstNBI, AseI, BmrI, BspDI, ClaI, HincII, MspI, PsiI, SpeI, AsiSI, BmtI, BspEI, CspCI, HindIII, MwoI, PspGI, SphI, AvaI, BpmI, BspHI, CviAII, Hinfl, NaeI, PspOMI, SspI, Avail, Bpu10I, BspMI, DdeI, HinP1I, NarI, PspXI, StuI, AvrII, BpuEI, BsrBI, DpnI, HpaI, NciI, PstI, StyD4I, BaeI, BsaAI, BsrDI, DpnII, HpaII, NcoI, PvuI, and StyI.

15. A method for determining sequence information of a small RNA molecule, comprising:
constructing a sequencing library based on a small RNA molecule sample by the method according to any one of Claims 1 to 14; sequencing the sequencing library to obtain a sequencing result; and determining the sequence information of the small RNA molecule based on the sequencing result.

16. A kit for constructing an RNA sequencing library, comprising:
an RNA 3' terminus connecting module, comprising a 3' end linker of an RNA molecule; and
an RNA 5' terminus connecting module, comprising a 5' end linker of the RNA molecule;
wherein both the 5' end linker and the 3' end linker contain a partial sequence of a recognition site of a DNA endonuclease, and when a self-linking product of the 5' end linker and the 3' end linker is formed, a complete sequence of the recognition site is formed at the junction.

17. The kit according to Claim 16, wherein the 5' end of the 3' end linker carries at least one, at least two, at least three, or at least four consecutive nucleotides of the 3' end of the recognition site of the DNA endonuclease; optionally, the 5' end of the 3' end linker has an adenylation modification, and the 3' end has a dideoxy modification; and optionally, the 3' end linker is a DNA linker.

18. The kit according to any one of Claims 16 to 17, wherein the 3' end of the 5' end linker carries at least one, at least two, at least three, or at least four consecutive nucleotides of the 5' end of the recognition site of the DNA endonuclease; and optionally, the 5' end linker is a DNA linker.

19. The kit according to any one of Claims 16 to 18, wherein neither the 5' end linker nor the 3' end linker comprises the complete sequence of the recognition site of the DNA endonuclease.

20. The kit according to any one of Claims 16 to 19, wherein
(a) the RNA 3' terminus connecting module comprises a first ligase; and preferably, the first ligase is a truncated T4 RNA ligase 2 or a point mutant thereof; and/or
(b) the RNA 5' terminus connecting module comprises a second ligase, and preferably, the second ligase is a T4 RNA ligase 1.

21. The kit according to any one of Claims 16 to 20, further comprising an enzyme cleavage module, wherein the enzyme cleavage module comprises the DNA endonuclease; optionally, the enzyme cleavage module further comprises an enzyme capable of specifically hydrolyzing an RNA in a DNA-RNA heterozygous chain; and optionally, the enzyme capable of specifically hydrolyzing the RNA in the DNA-RNA heterozygous chain is RNase H.

22. The kit according to any one of Claims 16 to 21, further comprising an extension module, wherein the extension module comprises an elongase for extending an RNA molecule connected with the 3' end linker and the 5' end linker; and preferably, the extension module and the enzyme cleavage module can be integrated into one module.

23. The kit according to Claim 22, wherein the elongase comprises a reverse transcriptase and/or Bst polymerase; and preferably, the reverse transcriptase is an MMLV reverse transcriptase.

24. The kit according to any one of Claims 16 to 23, further comprising an amplification module, wherein the amplification module comprises an enzyme that is required for DNA amplification and can be used for amplifying a product of the enzyme cleavage module.

25. The kit according to any one of Claims 16 to 24, wherein the RNA is a small RNA; and preferably, the small RNA has a length of 15-200 nt.

26. The kit according to any one of Claims 16 to 25, wherein the DNA endonuclease can effectively cleave a DNA double strand containing the sequence of the recognition site thereof, but has low activity for a DNA single strand containing the sequence of the recognition site.

27. The kit according to Claim 26, wherein a cleavage efficiency of the DNA endonuclease for the DNA single strand containing the sequence of the recognition site thereof is at most 1/10, at most 1/100, at most 1/1000, or at most 1/10000 of a cleavage efficiency for a corresponding DNA:DNA double strand, the DNA:DNA double strand contains the DNA single strand of the same sequence and a DNA single strand complementary thereto; and preferably, the DNA endonuclease is inactive, or has no detectable activity, for the DNA single strand containing the sequence of the recognition site thereof.

28. The kit according to any one of Claims 16 to 27, wherein the DNA endonuclease is a double-stranded DNA endonuclease; and preferably, the DNA endonuclease is selected from the group of: AatII, BamHI, BsaBI, BsrFI, DraI, HphI, NdeI, Paul, PvuII, SwaI, Acc65I, BanI, BsaHI, BsrGI, DraIII, Hpy188I, NgoMI, RsaI, TaqI, AccI, BanII, BsaI, BsrI, DrdI, Hpy188III, NheI, RsrII, TfiI, AciI, BbsI, BsaJi, BssHI, BssHII, EaeI, Hpy99I, NlaIII, SacI, TliI, AclI, BbvCI, BsaWI, BssKI, EagI, HpyCH4III, NlaIV, SacII, TseI, AcuI, BbvI, BsaXI, BssSI, EarI, HpyCH4IV, NotI, SalI, Tsp45I, AfeI, BccI, BseRI, BstAPI, EciI, HpyCH4V, NruI, SapI, Tsp509I, AflII, BceAI, BseYI, BstBI, EcoNI, KasI, NsiI, Sau3AI, TspRI, AflIII, BcgI, BsgI, BstEII, EcoO109I, KpnI, NspI, Sau96I, Tth111I, AgeI, BciVI, BsiEI, BstF5I, EcoRI, MboI, PacI, SbfI, XbaI, AhdI, Bell, BsiHKAI, BstNI, EcoRV, MboII, PaeR7I, ScaI, XcmI, AleI, BfaI, BsiWI, BstUI, FatI, MfeI, PciI, ScrFI, XhoI, AluI, BfrBI, BsiI, BstXI, Paul, MluI, PflFI, SexAI, XmaI, AlwI, BfuAI, BsmAI, BstYI, Fnu4HI, MlyI, PflMI, SfaNI, XmnI, AlwNI, BfuCI, BsmBI, BstZ17I, FseI, MmeI, PhoI, SfcI, ZraI, ApaI, BglI, BsmFI, Bsu36I, FspI, MnlI, PleI, SfoI, ApaLI, BglII, BsmI, BtgI, HaeII, MscI, PmeI, SgrAI, Nb.BbvCI, ApeKI, BlpI, BsoBI, BtgZI, HaeIII, MseI, PmlI, SmaI, Nt.BbvCI, ApoI, Bme1580I, Bsp1286I, BtsI, HgaI, MsiI, PpuMI, SmlI, Nb.BsmI, AscI, BmgBI, BspCNI, Cac8I, HhaI, MspA1I, PshAI, SnaBI, Nt.BstNBI, AseI, BmrI, BspDI, ClaI, HincII, MspI, PsiI, SpeI, AsiSI, BmtI, BspEI, CspCI, HindIII, MwoI, PspGI, SphI, AvaI, BpmI, BspHI, CviAII, Hinfl, NaeI, PspOMI, SspI, Avail, Bpu10I, BspMI, DdeI, HinP1I, NarI, PspXI, StuI, AvrII, BpuEI, BsrBI, DpnI, HpaI, NciI, PstI, StyD4I, BaeI, BsaAI, BsrDI, DpnII, HpaII, NcoI, PvuI, and StyI.

29. A method for constructing a sequencing library for an RNA molecule in a sample, comprising the steps of:
(1) connecting a 3' end linker to the 3' terminus of the RNA molecule in the sample;
(2) connecting a 5' end linker to the 5' terminus of the RNA molecule in the sample connected with the 3' end linker;
(3) obtaining an extension product using an elongase and an extension primer based on the RNA molecule in the sample connected with the 3' end linker and the 5' end linker;
(4) treating the extension product using a DNA endonuclease; and
(5) performing PCR amplification on the extension product to obtain the sequencing library, wherein both the 5' end linker and the 3' end linker contain a partial sequence of a recognition site of the DNA endonuclease, and when a self-linking product of the 5' end linker and the 3' end linker is formed, a complete sequence of the recognition site is formed at the junction.

30. The method according to Claim 29, wherein the ligation reaction of step (1) uses a truncated T4 RNA ligase 2 or a point mutant thereof, and/or the ligation reaction of step (2) uses a T4 RNA ligase 1.

31. The method according to Claim 29 or 30, wherein the elongase used in step (3) is a reverse transcriptase and/or Taq enzyme; and preferably, the reverse transcriptase is an MMLV reverse transcriptase.

32. The method according to any one of Claims 29 to 31, wherein the RNA molecule in the sample is a small RNA molecule; and preferably, the small RNA molecule has a length of 15-200 nt.

33. The method according to any one of Claims 29 to 32, wherein a content of the RNA molecules in the sample is ≤ 100 pg, ≤ 200 pg, ≤ 500 pg, ≤ 1 ng, ≤ 2 ng, ≤ 5 ng, ≤ 10 ng, or ≤ 20 ng.

34. The method according to any one of Claims 29 to 33, wherein the content of the RNA molecules in the sample is ≥ 50 pg; and preferably, the content of the RNA molecules in the sample is 50 pg-20 ng.

35. A method for processing a sample containing a DNA-RNA-DNA:cDNA heterozygous chain and a DNA:cDNA double strand, wherein the method comprises adding a DNA endonuclease to the sample, and
a DNA:cDNA pairing region of the DNA:cDNA double strand contains a sequence of a recognition site of the DNA endonuclease;
optionally, an RNA:cDNA pairing region of the DNA-RNA-DNA:cDNA heterozygous chain contains the sequence of the recognition site;
optionally, the DNA-RNA-DNA:cDNA heterozygous chain is generated during construction of an RNA sequencing library;
optionally, the DNA:cDNA double strand comprises a self-linking product formed by a 5' end linker and a 3' end linker used during RNA sequencing library construction; and
optionally, when the self-linking product is formed, the sequence of the recognition site of the DNA endonuclease is formed at the junction of the 5' end linker and the 3' end linker.

36. The method according to any one of Claims 29 to 35, wherein the method can efficiently remove the self-linking product formed by the 5' end linker and the 3' end linker in a system; and preferably, the method can remove at least 50%, at least 60%, at least 70%, at least 80%, at least 90%, at least 95%, at least 98%, at least 99%, at least 99.5%, at least 99.8%, or at least 99.9% of the self-linking products in the system.

37. The method according to any one of Claims 29 to 36, wherein the 5' end of the 3' end linker carries at least one, at least two, at least three, or at least four consecutive nucleotides of the 3' end of the recognition site of the DNA endonuclease; optionally, the 5' end of the 3' end linker has an adenylation modification, and the 3' end has a dideoxy modification; and optionally, the 3' end linker is a DNA linker.

38. The method according to any one of Claims 29 to 37, wherein the 3' end of the 5' end linker carries at least one, at least two, at least three, or at least four consecutive nucleotides of the 5' end of the recognition site of the DNA endonuclease; and optionally, the 5' end linker is a DNA linker.

39. The method according to any one of Claims 29 to 38, wherein neither the 5' end linker nor the 3' end linker comprises the complete sequence of the recognition site of the DNA endonuclease.

40. The method according to any one of Claims 29 to 39, wherein the DNA endonuclease can effectively cleave a DNA double strand containing the sequence of the recognition site thereof, but has low activity for an RNA:DNA heterozygous chain containing the sequence of the recognition site.

41. The method according to Claim 40, wherein a cleavage efficiency of the DNA endonuclease for an RNA:cDNA heterozygous chain containing the sequence of the recognition site thereof is at most 1/10, at most 1/100, at most 1/1000, or at most 1/10000 of a cleavage efficiency for the corresponding DNA:cDNA double strand, and the DNA:cDNA double strand contains a cDNA of the same sequence and a DNA complementary thereto; and preferably, the DNA endonuclease is inactive, or has no detectable activity, for the RNA:cDNA heterozygous chain containing the sequence of the recognition site thereof.

42. The method according to any one of Claims 29 to 41, wherein the DNA endonuclease is a double-stranded DNA endonuclease; and preferably, the DNA endonuclease is selected from the group of: AatII, BamHI, BsaBI, BsrFI, DraI, HphI, NdeI, Paul, PvuII, SwaI, Acc65I, BanI, BsaHI, BsrGI, DraIII, Hpy188I, NgoMI, RsaI, TaqI, AccI, BanII, BsaI, BsrI, DrdI, Hpy188III, NheI, RsrII, TfiI, AciI, BbsI, BsaJi, BssHI, BssHII, EaeI, Hpy99I, NlaIII, SacI, TliI, AclI, BbvCI, BsaWI, BssKI, EagI, HpyCH4III, NlaIV, SacII, TseI, AcuI, BbvI, BsaXI, BssSI, EarI, HpyCH4IV, NotI, SalI, Tsp45I, AfeI, BccI, BseRI, BstAPI, EciI, HpyCH4V, NruI, SapI, Tsp509I, AflII, BceAI, BseYI, BstBI, EcoNI, KasI, NsiI, Sau3AI, TspRI, AflIII, BcgI, BsgI, BstEII, EcoO109I, KpnI, NspI, Sau96I, Tth111I, AgeI, BciVI, BsiEI, BstF5I, EcoRI, MboI, PacI, SbfI, XbaI, AhdI, Bell, BsiHKAI, BstNI, EcoRV, MboII, PaeR7I, ScaI, XcmI, AleI, BfaI, BsiWI, BstUI, FatI, MfeI, PciI, ScrFI, XhoI, AluI, BfrBI, BsiI, BstXI, Paul, MluI, PflFI, SexAI, XmaI, AlwI, BfuAI, BsmAI, BstYI, Fnu4HI, MlyI, PflMI, SfaNI, XmnI, AlwNI, BfuCI, BsmBI, BstZ17I, FseI, MmeI, PhoI, SfcI, ZraI, ApaI, BglI, BsmFI, Bsu36I, FspI, MnlI, PieI, SfoI, ApaLI, BglII, BsmI, BtgI, HaeII, MscI, PmeI, SgrAI, Nb.BbvCI, ApeKI, BlpI, BsoBI, BtgZI, HaeIII, MseI, PmlI, SmaI, Nt.BbvCI, ApoI, Bme1580I, Bsp1286I, BtsI, HgaI, MsiI, PpuMI, SmlI, Nb.BsmI, AscI, BmgBI, BspCNI, Cac8I, HhaI, MspA1I, PshAI, SnaBI, Nt.BstNBI, AseI, BmrI, BspDI, ClaI, HincII, MspI, PsiI, SpeI, AsiSI, BmtI, BspEI, CspCI, HindIII, MwoI, PspGI, SphI, AvaI, BpmI, BspHI, CviAII, Hinfl, NaeI, PspOMI, SspI, Avail, Bpu10I, BspMI, DdeI, HinP1I, NarI, PspXI, StuI, AvrII, BpuEI, BsrBI, DpnI, HpaI, NciI, PstI, StyD4I, BaeI, BsaAI, BsrDI, DpnII, HpaII, NcoI, PvuI, and StyI; and preferably, the double-stranded DNA endonuclease is not AvaII, AvrII, BanI, HaeIII, Hinfl, TaqI, or other enzymes that can cleave a DNA/RNA heterozygous chain under specific conditions.

43. A method for determining sequence information of a small RNA molecule, comprising:
constructing a sequencing library based on a small RNA molecule sample by the method according to any one of Claims 29 to 42; sequencing the sequencing library to obtain a sequencing result; and determining the sequence information of the small RNA molecule based on the sequencing result.

44. The kit according to any one of Claims 22 to 28, wherein the elongase comprises a reverse transcriptase and/or Taq enzyme; and preferably, the reverse transcriptase is an MMLV reverse transcriptase.

45. The kit according to any one of Claims 16 to 28 and 44, wherein the DNA endonuclease can effectively cleave the DNA double strand containing the sequence of the recognition site thereof, but has low activity for an RNA:DNA heterozygous chain containing the sequence of the recognition site.

46. The kit according to Claim 45, wherein a cleavage efficiency of the DNA endonuclease for an RNA:cDNA heterozygous chain containing the sequence of the recognition site thereof is at most 1/10, at most 1/100, at most 1/1000, or at most 1/10000 of a cleavage efficiency for the corresponding DNA:cDNA double strand, and the DNA:cDNA double strand contains a cDNA of the same sequence and a DNA complementary thereto; and preferably, the DNA endonuclease is inactive, or has no detectable activity, for the RNA:cDNA heterozygous chain containing the sequence of the recognition site thereof.

47. The kit according to any one of Claims 16 to 28 and 44 to 46, wherein the double-stranded DNA endonuclease is not Avail, AvrII, BanI, HaeIII, Hinfl, TaqI, or other enzymes that can cleave a DNA/RNA heterozygous chain under specific conditions.

48. A system for determining sequence information of an RNA molecule, comprising:
the kit according to any one of Claims 16 to 28 and 44 to 47;
a sequencing device, used for sequencing the sequencing library constructed for a sample by the kit to obtain a sequencing result of the sample; and
an analysis device, for analyzing the sequencing result, to determine the sequence information of the RNA molecule; wherein
preferably, the RNA molecule is a small RNA molecule.

49. Use of the method according to any one of Claims 1 to 15 and 29 to 43, or the kit according to any one of Claims 16 to 28 and 44 to 47, or the system according to Claim 48, in construction of an RNA sequencing library.

50. The use according to Claim 49, wherein the RNA sequencing library is selected from the group of: a plasma small RNA sequencing library, a CLIP library, an RIP library, an MeRIP library, and a GRO library.

51. A method for constructing a sequencing library, comprising the steps of:
(1) connecting a specific tag linker to the 3' terminus of an RNA molecule in a mixed sample of DNA and RNA molecules;
(2) obtaining a single-stranded DNA molecule using a reverse transcription primer based on the RNA molecule connected with the tag linker; and
(3) constructing a library for all DNA molecules in the sample.

52. A method for simultaneous sequencing of DNA and RNA molecules in a sample, comprising the steps of:
(1) connecting a specific tag linker to the 3' terminus of an RNA molecule in a mixed sample of DNA and RNA molecules;
(2) obtaining a single-stranded DNA molecule using a reverse transcription primer based on the RNA molecule connected with the tag linker;
(3) constructing a library for all DNA molecules in the sample; and
(4) sequencing the library, and using a sequence of the specific tag linker to distinguish the RNA molecules in the mixed sample.

53. The method according to Claim 51 or 52, wherein a ligase in the ligation reaction of step (1) is a truncated T4 RNA ligase 2 or a point mutant thereof.

54. The method according to any one of Claims 51 to 53, wherein a library construction method used in step (3) comprises:
(a) denaturing the DNA molecule into a single strand;
(b) connecting specific linkers to the 3' terminus and/or 5' terminus of the single strand obtained in step (a); and
(c) optionally, using an amplification primer for amplification to obtain an amplification product.

55. The method according to any one of Claims 51 to 53, wherein a library construction method used in step (3) comprises:
(A) extending the single-stranded DNA molecule obtained in step (2) into a double-stranded DNA;
(B) connecting double-stranded linkers to one or both ends of the double-stranded DNA obtained in step (A); and
(C) optionally, using an amplification primer for amplification to obtain an amplification product.

56. The method according to Claim 54, wherein the library construction method used in step (3) further comprises:
(d) extending the product obtained in step (b) into a double-stranded DNA;
(e) connecting double-stranded linkers to one or both ends of the double-stranded DNA obtained in step (d); and
(f) optionally, using an amplification primer for amplification to obtain an amplification product.

57. The method according to any one of Claims 52 to 56, wherein a sequencing method used in step (4) comprises using next-generation high-throughput sequencing or third-generation sequencing.

58. The method according to any one of Claims 51 to 57, wherein the 5' end of the specific tag linker has an adenylation modification, and the 3' end has a dideoxy modification; and optionally, the specific tag linker is a DNA linker.

59. The method according to any one of Claims 51 to 58, wherein the ligase used in the ligation reaction in step (1) can connect the specific linker to the 3' terminus of the RNA molecule but cannot connect the specific linker to the 3' terminus of the DNA molecule.

60. A method for determining sequence information of DNA and RNA molecules in a mixed sample of the DNA and RNA molecules, comprising:
based on the mixed sample of the DNA and RNA molecules, constructing a sequencing library by the method according to any one of Claims 51 to 57 and performing sequencing, to obtain a sequencing result; and
based on the sequencing result, determining the sequence information of the DNA and RNA molecules in the mixed sample of the DNA and RNA molecules.

61. A method for determining sequence information of a DNA target region being transcribed and a synthesized RNA molecule in a transcription complex, comprising:
(i) obtaining a DNA and RNA sample of the region being transcribed;
(ii) constructing a sequencing library for the sample by the method according to any one of Claims 51 to 57 and performing sequencing, to obtain a sequencing result;
(iii) based on the sequencing result, determining the sequence information of the DNA target region being transcribed and the synthesized RNA molecule.

62. The method according to Claim 61, wherein step (i) comprises:
randomly fragmenting chromatin in the sample, and performing co-immunoprecipitation on the chromatin using an antibody for an RNA polymerase, to obtain the DNA and RNA sample of the region being transcribed.

63. The method according to Claim 62, wherein the randomly fragmented chromatin has an average length of 200 bp-500 bp.

64. The method according to Claim 62 or 63, wherein step (i) comprises degrading proteins using proteinase K after the co-immunoprecipitation.

65. The method according to any one of Claims 51 to 64, wherein the sample contains a cell-free DNA and cell-free RNA.

66. A kit for constructing a sequencing library of DNA and RNA molecules in the same sample, comprising:
an RNA terminus connecting module, which comprises a specific tag linker and is used for connecting the tag linker to the 3' terminus of the RNA molecule.

67. The kit according to Claim 66, further comprising:
a reverse transcription module, which comprises a reverse transcription primer and a reverse transcriptase and is used for generating a DNA molecule based on the RNA molecule connected with the tag linker.

68. The kit according to Claim 66 or 67, further comprising:
a DNA library construction module, which is used for constructing the DNA molecule in the sample into the sequencing library.

69. The kit according to any one of Claims 66 to 68, wherein the RNA terminus connecting module comprises a ligase; and preferably, the ligase is a truncated T4 RNA ligase 2 or a point mutant thereof.

70. The kit according to any one of Claims 51 to 69, wherein the 5' end of the specific tag linker has an adenylation modification, and the 3' end has a dideoxy modification; and optionally, the specific tag linker is a DNA linker.

71. A system for determining sequence information of DNA and RNA molecules, comprising:
the kit according to any one of Claims 66 to 70;
a sequencing device, used for sequencing the sequencing library constructed for a sample by the kit to obtain a sequencing result of the sample; and
an analysis device, for analyzing the sequencing result of the sample to obtain the sequence information of the DNA and RNA molecules.

72. Use of the method according to any one of Claims 51 to 65, or the kit according to any one of Claims 66 to 70, or the system according to Claim 71 in simultaneous sequencing of DNA and RNA molecules in the same sample.
